(19) Europäisches Patentamt / European Patent Office / Office européen des brevets

(11) EP 1 538 203 B1

(12) **EUROPEAN PATENT SPECIFICATION**

(45) Date of publication and mention of the grant of the patent:
**20.01.2010 Bulletin 2010/03**

(51) Int Cl.:
***C12N 9/48*** *(2006.01)*

(21) Application number: **04028526.4**

(22) Date of filing: **02.12.2004**

(54) **Recombinantly expressed carboxypeptidase B and purification thereof**

Rekombinante Carboxypeptidase B und ihre Aufreinigung

Carboxypeptidase B recombinante et sa purification

(84) Designated Contracting States:
**AT BE BG CH CY CZ DE DK EE ES FI FR GB GR HU IE IS IT LI LT LU MC NL PL PT RO SE SI SK TR**
Designated Extension States:
**LV**

(30) Priority: **05.12.2003 EP 03028101**

(43) Date of publication of application:
**08.06.2005 Bulletin 2005/23**

(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AT BE BG CH CY CZ DK EE ES FI FR GB GR HU IE IS IT LI LT LU MC NL PL PT RO SE SI SK TR**

(72) Inventors:
- **Glaser, Stephan, Dr.**
  **82402 Seeshaupt (DE)**
- **Geipel, Frank, Dr.**
  **82377 Penzberg (DE)**
- **Kirschbaum, Thomas, Dr.**
  **82377 Penzberg (DE)**
- **Rexer, Bernhard, Dr.**
  **82362 Weilheim (DE)**
- **Thalhofer, Johann-Peter, Dr.**
  **82362 Weilheim (DE)**
- **Mueller, Rainer, Dr.**
  **82377 Penzberg (DE)**
- **Giessel, Claudia**
  **83646 Bad Toelz (DE)**
- **Eckstein, Hellmut**
  **82362 Weilheim (DE)**
- **Wolf, Elvira**
  **83674 Gaissach (DE)**

(56) References cited:
**WO-A-01/51624** **WO-A-96/23064**
**DE-A- 19 915 938** **US-A- 5 948 668**

- **LI SU-XIA ET AL: "Cloning and expression of a new rat procarboxypeptidase B gene in Escherichia coli and purification of recombination carboxypeptidase B." PROTEIN AND PEPTIDE LETTERS, vol. 10, no. 6, December 2003 (2003-12), pages 581-590, XP009029264 ISSN: 0929-8665**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention pertains to the field of biotechnology. More specifically, the invention pertains to the production and further processing of an enzymatically inactive precursor of a protein with carboxypeptidase B activity. An aspect of the invention pertains to the activation and concomitant purification of the protein with carboxypeptidase B activity.

Carboxypeptidases are enzymes that hydrolyze C-terminal peptide bonds. The carboxypeptidase family includes metallo-, serine, and cysteine carboxypeptidases. According to their substrate specificity, these enzymes are referred to as carboxypeptidase A (cleaving aliphatic residues) or carboxypeptidase B (cleaving basic amino residues). Carboxypeptidase B (EC 3.4.17.2) is an enzyme that catalyzes hydrolysis of the basic amino acids, Lysine, Arginine, and Ornithine from the C-teminal position in polypeptides:

Peptidyl-L-Lysine(-L-basic amino acid) + $H_2O \rightarrow$ Peptide + L-Lysine(-L-basic amino acid)

The precursor of carboxypeptidase B is a zymogen or proenzyme in the pancreas of most vertebrates (reviewed by Folk J. Carboxypeptidase B, in: The Enzymes 3, P. Boyer, Academic Press, NY, 57, 1971). Carboxypeptidase B may function in the further degradation of products of tryptic digestion, for which it is specific, very much as carboxypeptidase A is specific for products of chymotrypsin. Carboxypeptidase B also has an esterase activity which is related to the metal content of the enzyme (Folk, J., and Gladner, J.: Biochim Biophys Acta (1961) 48, 139-47; Zisapel, N. and Sokolovsky, M, Eur J Biochem (1975) 54, 541-7).

A "zymogen" or a "proenzyme" is the inactive precursor of an enzyme. In other words, a zymogen or a proenzyme is the inactive precursor of a protein with enzymatic activity such as a protein with carboxypeptidase B activity. Many proteins with enzymatic activity are synthesized as such inactive precursors and are subsequently activated by cleavage of one or a few specific peptides bonds. Activation of enzymes and other proteins by specific proteolysis occurs frequently in biological systems. For example: (a) The fibrous protein collagen which is present in skin and bone is derived from procollagen which is a soluble precursor. (b) Some protein hormones are sythesized as inactive precursors. For example, insulin is derived from pro-insulin by proteolytic removal of a peptide. (c) The digestive enzymes that hydrolyze proteins are synthesized and secreted as zymogens in the stomach and pancreas. Carboxypeptidase B belongs to this group. (d) Blood clotting is mediated by a cascade of proteolytic activations that assures a rapid and amplified response to trauma.

In vivo the zymogen of carboxypeptidase B, that is to say "pro-carboxypeptidase B", is translated in pancreatic cells as a pre-protein termed "pre-pro-carboxypeptidase B".

Pre-pro-carboxypeptidase B comprises at its N-terminus a signal peptide. The amino acid sequence of pre-pro-carboxypeptidase B describes the primary translational product. The signal peptide directs pre-pro-carboxypeptidase B polypeptide to the secretory pathway of the pancreatic cell. During the secretion process the leader peptide is cleaved off proteolytically, thereby converting pre-pro-carboxypeptidase B to pro-carboxypeptidase B.

Pro-carboxypeptidase B is devoid of enzymatic activity. In vivo activation usually takes place in the small intestine. Trypsin cleaves pro-carboxypeptidase B, thereby generating an enzymatically inactive propeptide and the proteolytically active carboxypeptidase B enzyme.

As an example for pre-pro-carboxypeptidase, SEQ ID NO: 1 depicts the amino acid sequence of rat pre-pro-carboxypeptidase B as published by Clauser E. et al., J. Biol. Chem. 1988, Vol. 263, 17837-45. Accordingly, the first 13 amino acids of the pre-pro-carboxypeptidase B polypeptide represent the signal peptide and the following 95 amino acids constitute the propeptide. The activation product, i.e. the active rat carboxypeptidase B enzyme contains 307 amino acids with a calculated molecular weight of 35,228 Da.

Using trypsin pro-carboxypeptidase B can also be activated in vitro to form carboxypeptidase B. The co-formation of the propeptide and the enzyme moiety has been shown by analytical methods like SDS gel electrophoreses as well as reversed phase HPLC by Ventura et al. J. Biol. Chem. (1999) 274(28), 19925-33.

Activation experiments in vitro under various conditions have shown that while a proteolytically active carboxypeptidase B enzyme is readily generated, under non-denaturing conditions the propeptide remains non-covalently attached to a large number of carboxypeptidase B molecules. This could be shown when denaturing conditions were applied to the complex of the propeptide and the enzyme. Thus, non-denaturing purification methods and particularly anion exchange chromatography so far do not provide sufficient means to separate the non-covalently attached propeptide from the carboxypeptidase B enzyme.

Because of its high specificity for C-terminal basic amino acids, carboxypeptidase B has found wide use, e.g. in end-group analysis for sequence determination. However, of major economical importance are industrial applications which involve proteolytic processing. Particularly, carboxypeptidase B is used in production processes that lead to insulin for medical use and that involve proteolytic cleavage of pro-insulin.

Regarding insulin as a medical product a particular problem arises by virtue of the carboxypeptidase B propeptide that is non-covalently attached to the carboxypeptidase B enzyme. In said insulin production processes, subsequent to proteolytic cleavage of pro-insulin, the insulin fragment is purified under denaturing conditions. As a consequence, it is possible that the carboxypeptidase B propeptide co-purifies with insulin. In such a case separation of the carboxypeptidase B propeptide from the insulin molecule can be a laborious process which at the same time is likely to decrease the final yield of purified insulin.

Commercially available carboxypeptidase B purified from porcine pancreas may not be totally free of other proteolytic activities. Moreover, as it is the case for most animal-derived products, porcine carboxypeptidase B may contain infectious agents such as viruses, prions, or other biologically active components with deleterious potential for human health. It is therefore desired to obtain carboxypeptidase B from an alternative source. In addition, it is desired that carboxypeptidase B from the alternative source is substantially free of the propeptide.

It is known to the art that carboxypeptidase B can be produced recombinantly in transformed host organisms (e.g. Ventura et al. J. Biol. Chem. (1999) 274(28), 19925-33) and purified therefrom.

WO 01/51624 discloses recombinant expression of porcine pro-carboxypeptidase B in the methylotrophic yeast Pichia pastoris. Following activation of the zymogen by way of tryptic cleavage, a first purification step includes hydrophobic chromatography. Following the addition of soybean trypsin inhibitor, the activated enzyme is further purified using Q-sepharose chromatography. The purification method of WO 01/51624 involves multiple steps and there is concomitant loss of carboxypeptidase B product. In addition, there is the need to separate the soybean trypsin inhibitor as well as the carboxypeptidase B propeptide from the product.

DE 19 915 938 discloses recombinant expression of a Histidine-tagged human pro-carboxypeptidase B in the methylotrophic yeast Pichia pastoris. The product is purified using affinity chromatography and by virtue of the Histidine-tag. Subsequently, pro-carboxypeptidase B is activated using trypsin and the activation reaction is terminated by the addition of soybean trypsin inhibitor. By way of filtering through a membrane which excludes particles above a molecular weight of about 30,000 Da, carboxypeptidase B is separated from trypsin, the propeptide, the inhibitor as well as other fragments that were generated during the course of the purification procedure. However, as the molecular weight of carboxypeptidase B enzyme is about 37,000 Da, losses can be expected due to the filtration step, depending on the size distribution of the pores in the filter used. Moreover, as the activation step is performed on the dissolved zymogen there is the need to further separate the propeptide from the carboxypeptidase B enzyme.

In WO 96/23064 recombinant expression of rat pro-carboxypeptidase B in E.coli is described. Inclusion bodies of insoluble pro-carboxypeptidase B are produced. The zymogen is renatured and subsequently purified further. The activation step is performed on the dissolved zymogen and there is the need to further separate the propeptide from the carboxypeptidase B enzyme. Owing to the denaturation/renaturation steps the yield of carboxypeptidase B enzyme obtainable using this method is comparably low.

To the knowledge of the inventors, no specific method to separate activated carboxypeptidase B from the non-activated form is known.

The problem to be solved by the present invention is therefore to provide an alternative method to produce a protein with carboxypeptidase B activity from a zymogen, whereby non-covalent attachment of the propeptide to the protein with carboxypeptidase B activity is avoided. Another problem to be solved by the present invention is to provide an alternative method to separate the protein with carboxypeptidase B activity from the propeptide and from residual zymogen following the activation step. Another problem to be solved by the present invention is to provide an alternative method to separate under non-denaturing conditions the protein with carboxypeptidase B activity from the propeptide and from residual zymogen, following the activation step.

According to the invention a method is provided to produce a protein with carboxypeptidase B activity, comprising the steps of a) providing a vector comprising a nucleotide sequence which encodes a pre-protein consisting of the rat pro-carboxypeptidase B that is N-terminally fused to a Histidine-tag and a signal peptide, whereby optionally between the Histidine-tag and the signal peptide or between the Histidine-tag and rat carboxypeptidase B a spacer sequence is inserted; (b) transforming a microbial host organism with the vector; (c) cultivating the microbial host organism in a growth medium containing nutrients and a carbon source, whereby the microbial host organism expresses the pre-protein and secretes the Histidine-tagged pro-carboxypeptidase B into the growth medium; (d) immobilizing the secreted Histidine-tagged pro-carboxypeptidase B in the growth medium of step (c) on a particulate metal chelate affinity matrix capable of binding the Histidine-tag, and washing the particulate metal chelate affinity matrix, whereby the Histidine-tagged pro-carboxypeptidase B is immobilized; (e) incubating the particulate metal chelate affinity matrix with the immobilized Histidine-tagged pro-carboxypeptidase B of step (d) in a buffer containing trypsin, thereby cleaving proteolytically the pro-carboxypeptidase B moiety and releasing the protein with carboxypeptidase B activity into the liquid phase, whereby the Histidine-tagged propeptide moiety is immobilized; (f) separating the liquid phase containing the protein with carboxypeptidase B activity from the particulate metal chelate affinity matrix, whereby the Histidine-tagged propeptide moiety is immobilized on the particulate metal chelate affinity matrix ; and (g) purifying the protein with carboxypeptidase B activity from the liquid phase of step (f).

Certain terms are used with particular meaning, or are defined for the first time, in this description of the present invention. For the purposes of the present invention, the following terms are defined by their art-accepted definitions, when such exist, except that when those definitions conflict or partially conflict with the definitions set forth below. In the event of a conflict in definition, the meaning of the terms are first defined by the definitions set forth below.

Amino acid identification uses the the three-letter abbreviations as well as the single-letter alphabet of amino acids, i.e., Asp D Aspartic acid, Ile I Isoleucine, Thr T Threonine, Leu L Leucine, Ser S Serine, Tyr Y Tyrosine, Glu E Glutamic acid, Phe F PhenylAlanine, Pro P Proline, His H Histidine, Gly G Glycine, Lys K Lysine, Ala A Alanine, Arg R Arginine, Cys C Cysteine, Trp W Tryptophan, Val V Valine, Gln Q Glutamine, Met M Methionine, Asn N Asparagine. An amino acid at a particular position in an amino acid sequence is given by its three-letter abbreviation and a number. As an example, referring to the amino acid sequence of native rat pre-pro carboxypeptidase B of SEQ ID NO: 1, "His14" denotes the Histidine residue at amino acid position 14.

The term "comprising" is used in the description of the invention and in the claims to mean "including, but not necessarily limited to".

The term "pre-protein" refers to a primary translation product comprising at its N-terminus a signal peptide. In the context of the present invention the "pre-protein" is the precursor of zymogen or pro-enzyme, i.e. pro-carboxypeptidase B. The pro-enzyme results from post-translational processing of the pre-protein.

A "signal peptide" is a cleavable signal sequence of amino acids present in the pre-protein form of a secretable protein. Proteins transported across the cell membrane, i.e. "secreted", typically have an N- terminal sequence rich in hydrophobic amino acids about 15 to 30 amino acids long. Sometime during the process of passing through the membrane, the signal sequence is cleaved by a signal peptidase (Alberts, B., Johnson, A., Lewis, J., Raff, M., Roberts, K., Walter, P. (eds), Molecular Biology of the Cell, fourth edition, 2002, Garland Science Publishing). Many sources of signal peptides are well known to those skilled in the art and can include, for example, the amino acid sequence of the $\alpha$-factor signal peptide from Saccharomyces cerevisiae and the like. In general, the pre-protein N-terminus of essentially any secreted protein is a potential source of a signal peptide suitable for use in the present invention. A signal peptide can also be bipartite comprising two signal peptides directing the pre-protein to a first and a second cellular compartment. Bipartite signal peptides are cleaved off stepwise during the course of the secretory pathway. A preferred example therefor is the prepro peptide of the $\alpha$-factor from Saccharomyces cerevisiae (Waters et al., J. Biol. Chem. 263 (1988) 6209-14). An even more preferred example is the amino acid sequence encoded by the nucleotide sequence from position 1 to position 255 in SEQ ID NO: 3.

Pre-proteins with a secretory N-terminal signal peptide are directed to enter the "secretory pathway". The secretory pathway comprises the processes of post-translational processing and finally results in secretion of pro-carboxypeptidase B. In the present document it is understood that proteins secreted by methylotrophic yeast strains have passed through the secretory pathway.

The term "post-translational processing" denotes the modification steps a pre-protein is subjected to, in order result in a protein in a cellular or extracellular compartment. In the context of the present invention post-translational processing of pre-pro-carboxypeptidase B results in secreted pro-carboxypeptidase B.

Enzymatic proteolytic cleavage of pro-carboxypeptidase B, e.g. by trypsin, is referred to as "activation". It is well known to the skilled artisan that the molecular events leading to activation depend on proteolytic processing. Trypsin cleaves pro-carboxypeptidase B, thereby generating an enzymatically inactive "propeptide" or "propeptide moiety" and a proteolytically active "enzyme moiety", that is to say carboxypeptidase B. By way of example, enzyme moiety of rat pro-carboxypeptidase B, that is to say the "pro-carboxypeptidase B moiety" of the rat procarboxypeptidase B polypeptide is the polypeptide given by the amino acid sequence from position 191 to position 497 in SEQ ID NO: 4.

The propeptide of a pro-carboxypeptidase B usually comprises about 95 amino acids. It is also known that proteolytic cleavage of pro-carboxypeptidase B by trypsin not only activates carboxypeptidase B but also may form tryptic fragments of carboxypeptidase B.

A "methylotrophic yeast" is defined as a yeast that is capable of utilising methanol as its carbon source. The term also comprises laboratory strains thereof. In case a methylotrophic yeast strain is auxotrophic and because of this needs to be supplemented with an auxillary carbon-containing substance such as, e.g. Histidine in the case of a methylotrophic yeast strain unable to synthesise this amino acid in sufficient amounts, this auxillary substance is regarded as a nutrient but not as a carbon source.

A "vector" is defined as DNA which can comprise, i.e. carry and maintain the DNA fragment of the invention, including, for example, phages and plasmids. These terms are understood by those of skill in the art of genetic engineering. The term "expression cassette" denotes a nucleotide sequence encoding a pre-protein, operably linked to a promoter and a terminator. As for vectors containing an expression cassette, the terms "vector" and "expression vector" are synonyms.

"Transformation" means introducing DNA into an organism so that the DNA is replicable, either as an extrachromosomal element or by chromosomal integration.

The term "episome" denotes a unit of genetic material composed of a series of genes that sometimes has an independent existence in a host cell and at other times is integrated into a chromosome of the cell, replicating itself along with the chromosome. An example for an episome is the vector of Figure 1.

The term "expression" and the verb "to express" denote transcription of DNA sequences and/or the translation of the transcribed mRNA in a host organism resulting in a pre-protein, i.e. not including post-translational processes.

A nucleotide sequence "encodes" a peptide or protein when at least a portion of the nucleotide sequence, or its complement, can be directly translated to provide the amino acid sequence of the peptide or protein, or when the isolated nucleotide sequence can be used, alone or as part of an expression vector, to express the peptide or protein in vitro, in a prokaryotic host cell, or in a eukaryotic host cell.

All nucleotide sequences are written in the direction from the 5' (stands for prime) end to the 3' end also referred to as 5' to 3'.

A "promoter" is a regulatory nucleotide sequence that stimulates transcription. These terms are understood by those of skill in the art of genetic engineering. Like a promoter, a "promoter element" stimulates transcription but constitutes a sub-fragment of a larger promoter sequence.

The term "operably linked" refers to the association of two or more nucleic acid fragments on a single vector so that the function of one is affected by the other. For example, a promoter is operably linked with a coding sequence, i.e. a nucleotide sequence encoding a protein or a pre-protein, when it is capable of affecting the expression of that coding sequence, i.e., that the coding sequence is under the transcriptional control of the promoter.

A "peptide bond" is a covalent bond between two amino acids in which the $\alpha$-amino group of one amino acid is bonded to the alpha-carboxyl group of the other amino acid.

## Detailed description of the invention

A first embodiment of the invention is a method to produce a protein with carboxypeptidase B activity, comprising the steps of a) providing a vector comprising a nucleotide sequence which encodes a pre-protein consisting of the rat pro-carboxypeptidase B that is N-terminally fused to a Histidine-tag and a signal peptide, whereby optionally between the Histidine-tag and the signal peptide or between the Histidine-tag and rat carboxypeptidase B a spacer sequence is inserted; (b) transforming a microbial host organism with the vector; (c) cultivating the microbial host organism in a growth medium containing nutrients and a carbon source, whereby the microbial host organism expresses the pre-protein and secretes the Histidine-tagged pro-carboxypeptidase B into the growth medium; (d) immobilizing the secreted Histidine-tagged pro-carboxypeptidase B in the growth medium of step (c) on a particulate metal chelate affinity matrix capable of binding the Histidine-tag, and washing the particulate metal chelate affinity matrix,whereby the Histidine-tagged pro-carboxypeptidase B is immobilized; (e) incubating the particulate metal chelate affinity matrix with the immobilized Histidine-tagged pro-carboxypeptidase B of step (d) in a buffer containing trypsin, thereby cleaving proteolytically the pro-carboxypeptidase B moiety and releasing the protein with carboxypeptidase B activity into the liquid phase, whereby the Histidine-tagged propeptide moiety is immobilized; (f) separating the liquid phase containing the protein with carboxypeptidase B activity from the particulate metal chelate affinity matrix, whereby the Histidine-tagged propeptide moiety is immobilized on the particulate metal chelate affinity matrix ; and (g) purifying the protein with carboxypeptidase B activity from the liquid phase of step (f).

When constructing a vector to be used for transformation of a microbial host strain, e.g. a methylotrophic yeast strain, some molecular cloning techniques may require the addition of a linker or spacer sequence to a nucleotide sequence that encodes a functional element of the pre-protein. A spacer sequence may also be generated, e.g., when a DNA fragment comprising the coding sequence of a functional element of the pre-protein is excised from a cloning vector using a restriction endonuclease generating a fragment that is longer than the coding sequence. Other reasons why linker or spacer nucleotide sequences may occur are possible. Nucleotide sequences encoding functional elements of the pre-protein in this regard encode (a) the signal peptide, (b) the Histidine-tag and (c) the pro-carboxypeptidase B moiety. Thus, owing to the presence of such a linker nucleotide sequence, additional amino acids, i.e. a "spacer sequence", may be inserted at the junctions of any two functional elements of the pre-protein. An example therefor is the spacer sequence consisting of a Serine residue and an Alanine residue at amino acid positions 86 an 87 in SEQ ID NO: 3 and SEQ ID NO: 4. Preferred in the pre-protein of the invention are spacer sequences consisting of not more than four amino acid residues, More preferred are spacer sequences consisting of two amino acid residues. The insertion of a linker nucleotide sequence encoding a spacer sequence is "optional" in the sense that said insertion may facilitate the joining of the separate nucleotide sequences encoding two functional elements, in order to form a nucleotide sequence encoding the pre-protein or a precursor thereof.

A Histidine-tag is an amino acid sequence containing preferably 6 consecutive Histidines. As the Histidines represent the essential portion, there are few additional amino acids comprised in the Histidine-tag moiety. Importantly, the Histidine-tag used in the present invention does not add another relevant trypsin cleavage site to the secreted protein. Thus, the trypsin cleavage site of the zymogen remains the preferred cleavage site under the conditions applied during on-column activation of immobilized Histidine-tagged pro-carboxypeptidase B. Upon on-column activation of the immobilized Histidine-tagged pre-carboxypeptidase B the propeptide remains bound to the particulate metal chelating affinity matrix via the Histidine-tag.

Purification of secreted Histidine-tagged pro-carboxypeptidase B is facilitated by immobilized metal affinity chromatography. This method is a widely employed method to purify recombinant proteins containing a short affinity-tag consisting of histidine residues (Histidine-tag). Immobilized metal-affinity chromatography (described by Porath, J. et al., Nature 258 (1975) 598-599) is based on the interaction between a transition metal ion ($Co^{2+}$, $Ni^{2+}$, $Cu^{2+}$, $Zn^{2+}$) immobilized on a particulate metal chelating affinity matrix and specific amino acid side chains. Histidine is the amino acid that exhibits the strongest interaction with immobilized metal ion matrices, as electron donor groups on the histidine imidazole ring readily form coordination bonds with the immobilized transition metal. Peptides containing sequences of consecutive histidine residues are efficiently retained on particulate metal chelating affinity. Following washing of the matrix material, peptides containing histidine sequences such as a Histidine-tag can be easily eluted by either adjusting a low (acidic) pH of the column buffer or by adding free imidazole.

The method to purify proteins with histidine residues was first described in by Hochuli, E. et al., J. Chromatogr. 411 (1987) 177-184. The document describes a nitrilotriacetic acid (NTA) adsorbent for metal-chelate affinity chromatography. The NTA resin forms a quadridentate chelate and is especially suitable for metal ions with coordination numbers of six, since two valencies remain for the reversible binding of biopolymers. Dihydrofolate reductase with a Histidine-tag was successfully purified with $Ni^{2+}$ -NTA matrices as described by Hochuli, E. et al., Bio/Technology 6 (1988) 1321-1325. The purification efficiency of this system was dependent on the length of the Histidine-tag and the solvent system. While the system worked efficiently with His6-tagged proteins under denaturing conditions, His3-tagged proteins were efficiently purified under physiological conditions. However, His6-tagged proteins can be bound to $Ni^{2+}$ -NTA matrices under non-denaturing conditions in low- or high-salt buffers. After binding, the target protein can be eluted by an imidazole gradient from 0.8 to 250 mM. Washing with a low concentration of imidazole (e.g. 0.8 mM) can be used to reduce unspecific binding of host proteins with histidines.

In the present invention, the immobilization step is preferably performed in the presence of imidazole. Under these conditions, unspecific binding of other Histidine-containing proteins has been found to be reduced. Preferred concentrations of imidazole in this regard are in the range between 0.01 mM and 1 mM.

Another material that has been developed to purify Histidine-tagged proteins is TALON. It consists of a $Co^{2+}$-carboxylmethylaspartate ($Co^{2+}$-CMA), which is coupled to a solid-support resin. TALON has been reported to exhibit less unspecific protein binding than the $Ni^{2+}$-NTA resin, resulting in higher elution product purity (Chaga, G. et al., Biotechnol. Appl. Biochem. 29 (1999) 19-24; Chaga, G. et al., J. Chromatogr. A 864 (1999) 257-256).

Histidine-tags are commonly placed on either the N- or the C-terminus of recombinant proteins. Optimal placement of the tag is protein-specific. Purification using Histidine tags has been carried out successfully using a number of expression systems including bacteria (Chen, B.P. and Hai, T., Gene 139(1994) 73-75; Rank, K.B. et al., Protein Expr. Purif. 22 (2001) 258-266), yeast (Borsing, L. et al. Biochem. Biophys. Res. Commun. 240 (1997) 586-590; Kaslow, D.C. and Shiloach, J., Bio/Technology 12 (1994) 494-499), mammalian cells (Janknecht, R. et al. Proc. Natl. Acad. Sci. USA 88 (1991) 8972-8976; Janknecht, R. and Nordheim, A., Gene 121 (1992) 321-324), and baculovirus-infected insect cells (Kuusinen, A. et al., Eur. J. Biochem. 233 (1995) 720-726; Schmidt, M. et al. Protein Expr. Purif. 12 (1998) 323-330). More than 100 structures of Histidine-tagged proteins have been deposited in the Protein Data Bank. Proteins with a Histidine-tag may vary slightly as far as their mosaicity and diffraction compared to the native protein (Hakansson, K. et al. Acta Crystallogr. D Biol. Crystallogr. 56 (2000) 924-926). In principle, it cannot be excluded that the Histidine-tag may interfere with protein activity (Wu, J. and Filutowicz, M., Acta Biochim. Pol. 46 (1999) 591-599), although the relatively small size and charge of the Histidine-tag ensure that protein activity is rarely affected. Moving the Histidine-tag to the opposite terminus (Halliwell, C.M. et al., Anal Biochem. 295 (2001) 257-261) or carrying out the purification under denaturing conditions often solves this problem.

The secreted Histidine-tagged pro-carboxypeptidase B in the growth medium is immobilized on a particulate metal chelate affinity matrix capable of binding a Histidine tag. For instance, the Histidine-tagged pro-carboxypeptidase B can be adsorbed to metal ions immobilized on a metal-chelating resin. As described above, such resins are well known to the skilled artisan. A preferred particulate metal chelate affinity matrix is chromatography material that is coated with nickel-nitrilotriacetic acid (Ni-NTA). In this regard, the person skilled in the art is aware of EP 0 253 303, EP 0 282 042, and EP 1 069 131. Qiagen commercializes the QIAexpress purification system which can be used for the immobilization step. The same company provides an expression vector (pQE) that can be used to produce Histidine-tagged fusion polypeptides. In this regard, the person skilled in the art is also aware of US 5,284,933 and US 5,310,663.

Purification of protein with a metal center may require especially adapted methods for purification because the metal can be absorbed by the NTA. Purification under anaerobic conditions may also require especially adapted methods for purification because $Ni^{2+}$-NTA is reduced. Nevertheless, purification of pre-proteins with a Histidine-tag is one of the the most commonly used method.

Carboxypeptidase B contains $Zn^{2+}$ as a cofactor. For this reason, instead of using $Ni^{2+}$-NTA as a matrix for chromatographic purification, a metal chelating affinity matrix loaded with $Zn^{2+}$ is preferred. Thereby any accidental and unwanted replacement of the $Zn^{2+}$ cofactor by $Ni^{2+}$ is avoided.

An even more preferred particulate metal chelate affinity matrix is therefore $Zn^{2+}$- loaded (i.e. $Zn^{2+}$ ions immo-

bilized on) StreamlineTM Chelating adsorbent (Amersham Biosciences). StreamlineTM adsorbents are based on agarose. The macroporous structure of the highly cross-linked agarose matrices combines good binding capacities for large molecules, such as proteins, with high chemical and mechanical stability. High mechanical stability is an important property of a matrix to be used in expanded bed chromatography to reduce the effects of attrition when particles are moving freely in the expanded bed. Particles made only of organic material have limited density and would need to have very large diameters for the high sedimentation velocity required. Such large particle diameters result in long diffusional path lengths, which cause considerable mass transfer resistance, counteracting productivity. StreamlineTM adsorbents are therefore based on a composite particle containing an inert core material that is denser than organic materials. Such particles can be designed so that their sedimentation velocity is high also at a reasonable particle size.

Expanded bed adsorption is a single pass operation in which desired proteins are purified from crude, particulate containing feed-stock without the need for separate clarification, concentration and initial purification. The expansion of the adsorbent bed creates a distance between the adsorbent particals, i.e. increased voidage (void volume fraction) in the bed, which allows for unhindered passage of cells, cell debris and other particulates during application of crude feed to the column.

The particulate metal chelate affinity matrix (such as the StreamlineTM) adsorbent is expanded and equilibrated by applying an upward liquid flow to the column. A stable fluidized bed is formed when the adsorbent particles are suspended in equilibrium due to the balance between particle sedimentation velocity and upward liquid flow velocity. The column adaptor is positioned in the upper part of the column during this phase. Crude, unclarified feed, i.e. culture medium containing secreted Histidine-tagged pro-carboxypeptidase B and the microbial host organism, is applied to the expanded bed with the same upward flow as used during expansion and equilibration. Histidine-tagged pro-carboxypeptidase B is immobilized by way of binding with the Histidine-tag to the adsorbent while cell debris, cells, particulates and contaminants pass through unhindered. Weakly bound material, such as residual cells, cell debris and other type of particulate material, is washed out from the expanded bed using upward liquid flow. When the weakly retained material has been washed out from the bed the captured Histidine-tagged pro-carboxypeptidase B is activated by on-column cleavage with trypsin, whereby the upward flow mode is maintained. Carboxypeptidase B is released and can be washed from the column. The flow-through contains the target protein, increased in concentration, clarified, partly purified, and ready for further purification by packed bed chromatography.

How to use a preferred particulate metal chelate affinity matrix, i.e. StreamlineTM Chelating adsorbent (Amersham Biosciences) and how to set up, optimize and perform expanded bed chromatography is further detailed in in the Pharmacia Biotech manual "Expanded bed adsorption, principles and methods" (ISBN 91-630-5519-8). General instructions how to regenerate the column are also described therein.

A large number of microbial host organisms can be used in the present invention. The main requirement is that the microbial host organism is engineered and cultured such that it secretes Histidine-tagged pro-carboxypeptidase B into the growth medium. In a preferred embodiment the microbial host organism is a prokaryotic organism. In this regard, the skilled artisan is well aware of commercially available bacterial systems for recombinant expression and secretion which are based on bacteria such as E. coli, Bacillus sp., Staphylococcus sp.. In a more preferred embodiment the microbial host organism is a microbial eukaryotic organism. Very much preferred is a yeast species. In an even more preferred embodiment, the microbial organism is a methylotrophic yeast strain.

Thus, in another embodiment of the invention, Histidine-tagged rat pro-carboxypeptidase B is produced by recombinant means using methylotrophic yeast as a non-animal host organism. Methylotrophic yeasts have the biochemical pathways necessary for methanol utilization and are classified into four genera, based upon cell morphology and growth characteristics: Hansenula, Pichia, Candida, and Torulopsis. The most highly developed methylotrophic host systems utilize Pichia pastoris (Komagataella pastoris) and Hansenula polymorpha (Pichia angusta).

Expression of heterologous proteins in yeast is described in US 5,618,676, US 5,854,018, US 5,856,123, and US 5,919,651.

Yeast organisms produce a number of proteins that are synthesized intracellularly but have a function outside the cell. These extracellular proteins are referred to as secreted proteins. Initially the secreted proteins are expressed inside the cell in the form of a precursor or a pre-protein containing an N-terminal signal peptide ensuring effective direction of the expressed product into the secretory pathway of the cell, across the membrane of the endoplasmic reticulum. The signal peptide is generally cleaved off from the desired product during translocation. Cleavage is effected proteolytically by a signal peptidase. A particular sub-sequence of amino acids of the signal peptide is recognised and cleaved by the signal peptidase. This sub-sequence is referred to as signal peptidase cleavage site. Once having entered the secretory pathway, the protein is transported to the Golgi apparatus. From the Golgi apparatus the proteins are distributed to the plasma membrane, lysosomes and secretory vesicles.

Secreted proteins are confronted with different environmental conditions as opposed to intracellular proteins. Part of the processes of the secretory pathway is to stabilise the maturing extracellular proteins. Therefore, pre-proteins that are passed through the secretory pathway of yeast undergo specific posttranslational processing. For example, processing can comprise the generation of disulfide bonds to form intramolecular cross-links. Moreover, certain amino acids of the protein can be glycosylated.

Several approaches have been suggested for the expression and secretion in yeast of proteins heterologous to yeast. EP 0 116 201 describes a process by which proteins heterologous to yeast are transformed by an expression vector harboring DNA encoding the desired protein, a signal peptide and a peptide acting as a signal peptidase cleavage site. A culture of the transformed organism is prepared and grown, and the protein is recovered from culture media. For use in yeast cells a suitable signal peptide has been found to be the $\alpha$-factor signal peptide from Saccharomyces cerevisiae (US 4,870,008).

During secretion, the yeast enzyme KEX-2 is the signal peptidase which recognizes a Lysine-Arginine sequence as its cleavage site in the pre-protein. KEX-2 cleaves at the junction to the sequence of the desired protein. As a result, the desired gene product is released and free of the leader portions, i.e. the signal peptide of the pre-protein. KEX-2 endoprotease was originally characterised in Saccharomyces yeast where it specifically processes the precursor of mating type $\alpha$-factor and a killer factor (Julius, D., et al., Cell 37 (1984) 1075-1089). Methylotrophic yeast species such as Pichia pastoris share the KEX-2-type protease (similar role and function) with Saccharomyces cerevisiae (Werten, M.W., et al., Yeast 15 (1999) 1087-1096).

A well-established methylotrophic yeast species exemplarily described as host for high-level recombinant protein expression is Pichia pastoris (US 4,683,293, US 4,808,537, US 4,812,405, US 4,818,700, US 4,837,148, US 4,855,231, US 4,857,467, US 4,879,231, US 4,882,279, US 4,885,242, US 4,895,800, US 4,929,555, US 5,002,876, US 5,004,688, US 5,032,516, US 5,122,465, US 5,135,868, US 5,166,329, WO 00/56903). In the absence of glucose, Pichia pastoris uses methanol as a carbon source which at the same time is a hallmark of a methylotrophic organism. The alcohol oxidase (AOX1) promoter given in SEQ ID NO: 5 controls expression of alcohol oxidase, which catalyses the first step in methanol metabolism. Typically, 30% of the total soluble protein in methanol- induced cells is alcohol oxidase. Several Pichia expression vectors carry the AOX1 promoter and use methanol to induce high-level expression of desired heterologous proteins. Expression constructs also integrate into the Pichia pastoris genome, creating a transformed and genetically stable host.

Using an expression vector encoding a heterologous pre-protein comprising a signal peptide or a signal peptide with a signal peptidase cleavage site, and a desired protein, methylotrophic yeast strains such as Pichia pastoris strains can be manipulated in order to secrete the desired product into the growth medium from where the secreted protein can be purified. It may be advantageous to produce nucleotide sequences encoding the pre-protein possessing a substantially different codon usage.

Regarding the encoded pro-carboxypeptidase B polypeptide, the nucleotide sequence comprised in SEQ ID NO: 3 is different from previously published nucleotide sequences such as SEQ ID NO: 1 because of the degeneracy of the genetic code. The nucleotide sequence of SEQ ID NO: 3 from position 286 to position 1497 encodes the same polypeptide as the nucleotide sequence of SEQ ID NO: 1 from position 40 to position 1248. However, SEQ ID NO: 3 incorporates two amino acid exchanges, i.e. Lys201Asn and Arg329Asp. These amino acid exchanges are also documented in WO 96/23064. The term "degenerate code" indicates that in the genetic code a particular amino acid can be coded by two or more different codons. Degeneracy occurs because of the fact that of the 64 possible base triplets, 3 are used to code the stop signals, and the other 61 are left to code for only 20 different amino acids.

Thus, codons may be selected to increase the rate at which expression of the pre-protein occurs in a particular yeast expression host in accordance with the frequency with which particular codons are utilized by the host. Other reasons for substantially altering the nucleotide sequence encoding the pre-protein, without altering the encoded amino acid sequence, include the production of RNA transcripts having more desirable properties, such as a greater half-life, than transcripts produced from the naturally occurring sequence. The nucleotide sequence of SEQ ID NO: 3 is an example for a coding sequence that has been optimized in this fashion.

Using a vector comprising the nucleotide sequence encoding the pre-protein that is competent for expression, i.e. operably linked to a promoter or promoter element and to a terminator or terminator element, as well as to sequences required for efficient translation, the host organism is transformed with the vector and transformants are selected. Transformants are then analyzed with respect to the yield of recombinant protein secreted into the growth medium. Transformants secreting the highest quantities of recombinant protein are selected. Thus, transformants secreting the highest amounts of Histidine-tagged pro-carboxypeptidase B are selected.

On the one hand, expression yield is dependent on proper targeting of the desired product, that is to say targeting the pre-protein to the secretory pathway of yeast by means of the signal peptide. An example for a signal peptide is the $\alpha$-factor signal peptide from Saccharomyces cerevisiae encoded in SEQ ID NO: 3 from position 1 to position 255. On the other hand, expression yield can be increased by increasing the dosage of the gene encoding the desired product, i.e. the copy number of the expression construct in the host organism is amplified. One way to accomplish this is by multiple transformation of an expression vector encoding the desired product. Another way is to introduce the gene encoding the desired product into the host organism using a first and a second expression vector, whereby the second expression vector is based on a selectable marker which differs from the selectable marker used in the first expression vector. The second expression vector encoding the same desired product can even be introduced when the host organism already carries multiple copies of a first expression vector (US 5,324,639; Thill, G.P., et al., Positive and Negative Effects of Multi-Copy Integrated Expression in Pichia pastoris, International Symposium on the Genentics of Microorganisms 2 (1990), pp. 477-490; Vedvick, T., et al., J. Ind. Microbiol. 7 (1991) 197-201; Werten, M.W., et al., Yeast 15 (1999) 1087-1096).

Secretion of pro-carboxypeptidase B directs the recombinant protein to the extracytoplasmic space from where it diffuses into the growth medium. Thus, in a preferred embodiment of the invention the methylotrophic yeast is grown in liquid culture and secretes pro-carboxypeptidase B into the liquid growth medium, i.e. the liquid culture medium. This allows a very efficient separation of yeast biomass from the recombinant protein using, e.g. expanded bed chromatography techniques. As a result, pro-carboxypeptidase B purified from the yeast source organism can be freed efficiently from other not desired enzyme activities.

Regarding the vector with which, in a preferred embodiment, the methylotrophic yeast strain is transformed, the person skilled in the art is well aware of expression vectors that allow the construction of fusion polypeptides containing a so called "Histidine tag", "(poly)Histidine tag" or "Histidine-tag". In the preferred pre-protein of present invention, a Histidine-tag is fused to the N-terminus of the pro-carboxypeptidase B polypeptide. The Histidine-tag in total comprises six consecutive Histidine residues. The skilled artisan will note in this regard that the N-terminal amino acid of the pro-carboxypeptidase B polypeptide is a Histidine, too. Thus, in the annotated nucleotide sequence encoding the pre-protein there is an overlap of the fused coding sequences in that the codon for the last Histidine of the Histidine-tag also represents the first Histidine of the pro-carboxypeptidase B moiety.

After activation, i.e. the tryptic cleavage of the immobilized Histidine-tagged pro-carboxypeptidase B, the Histidine-tagged propeptide moiety remains bound to the particulate metal chelate affinity matrix. The activated carboxypeptidase B is released and separated in step (f) from the particulate metal chelate affinity matrix. Thus, separating particulate metal chelate affinity matrix from the liquid phase at the same time separates the propeptide from the carboxypeptidase B enzyme. Using this method non-covalent attachment of the propeptide to the carboxypeptidase B molecule is avoided.

It is preferred that the vector is comprising a nucleotide sequence which encodes a pre-protein consisting of the rat pro-carboxypeptidase B that is N-terminally fused to a Histidine-tag (Histidine-tag) and a signal peptide, whereby optionally between the Histidine-tag and the signal peptide a spacer sequence may be inserted. It is more preferred that the amino acid sequence of the rat pro-carboxypeptidase B is the amino acid sequence from position 14 to position 415 in SEQ ID NO: 3. It is also preferred that the signal peptide contains a signal peptidase cleavage site which is located adjacent to the Histidine-tag or adjacent to the spacer sequence. It is very much preferred that the amino acid sequence of the expressed pre-protein is the amino acid sequence in SEQ ID NO: 4. It is even more preferred that the nucleotide sequence encoding rat pro-carboxypeptidase B is the nucleotide sequence from position 286 to position 1,497 in SEQ ID NO: 3. It is even more preferred that the nucleotide sequence encoding the pre-protein is the nucleotide sequence in SEQ ID NO: 3. It is even more preferred that the nucleotide sequence encoding the pre-protein is operably linked to a promoter or promoter element.

To enable transcription of the nucleotide sequence encoding the pre-protein it is preferred that the nucleotide sequence encoding the pre-protein is operably linked to a promoter or promoter element. Very much preferred is a promoter or promoter element from Pichia pastoris, even more preferred is the Pichia pastoris AOX1 promoter given in SEQ ID NO: 5. It is also preferred that in addition the nucleotide sequence that encodes the pre-protein is operably linked with a terminator sequence that directs termination of transcription in the methylotrophic yeast strain. Very preferred is a terminator from Pichia pastoris, even more preferred is the Pichia pastoris AOX1 terminator.

It is further preferred that the vector is a plasmid capable of being replicated as an episome in the methylotrophic yeast strain. Thus, the preferred plasmid is a circular nucleic acid molecule that comprises an origin of replication directing replication of the episome in the methylotrophic yeast strain. Moreover, the plasmid comprises a selectable marker that is expressed in the methylotrophic yeast strain, whereby the selectable marker allows to select for the presence of the plasmid in the methylotrophic yeast strain. A very much preferred selectable marker is a Zeocin™ resistance gene, that is the native form or a genetically engineered variant of the Sh ble gene from Streptoalloteichus hindustanus (Drocourt, D., et al., Nucleic Acids Res. 18 (1990) 4009; Carmels, T., et al., Curr. Genet. 20 (1991) 309-314). Another very much preferred selectable marker confers resistance against aminoglycoside antibiotics such as Hygromycin and G418 (Southern, P.J., and Berg, P., J. Mol. Appl. Genet. 1 (1982) 327-341). An example for such a selectable marker is an aminoglycoside phosphotransferase gene.

It is further preferred that an artificial chromosome capable of being replicated in the methylotrophic yeast strain contains the vector. Thus, the preferred artificial chromosome is a linear nucleic acid molecule that comprises at least one origin of replication, a centromere and terminal telomeres, thereby controlling replication, integrity and mitotic/meiotic distribution of the artificial chromosome in the methylotrophic yeast strain. Moreover, the vector that is contained in the artificial chromosome comprises a selectable marker that is expressed in the methylotrophic yeast strain and that allows to select for the presence of the vector in the artificial chromosome that is replicated in the methylotrophic yeast strain. A very much preferred selectable marker is a Zeocin™ resistance gene, that is the native form or an artificial variant of the Sh ble gene from Streptoalloteichus hindustanus. Another very much preferred selectable marker confers resistance against aminoglycoside antibiotics such as Hygromycin and G418. An example for such a selectable marker is an aminoglycoside phosphotransferase gene.

It is even more preferred that a chromosome of the methylotrophic yeast strain contains the vector. It is very much preferred that the vector has a nucleotide sequence identical to a chromosomal sequence, thus allowing integration of the vector into the host chromosome by site-specific recombination. To this end, the Pichia pastoris AOX1 locus is even more preferred as a locus for integration the host chromosome by site-specific recombination. It is also very much preferred that, the vector comprises a selectable marker that is expressed in the methylotrophic yeast strain and that allows to select for the presence of the vector in the methylotrophic yeast strain. A very much preferred selectable marker is a Zeocin™ resistance gene, that is the native form or an artificial variant of the Sh ble gene from Streptoalloteichus hindustanus. Another very much preferred selectable marker confers resistance against aminoglycoside antibiotics such as Hygromycin and G418. An example for such a selectable marker is an aminoglycoside phosphotransferase gene.

The person skilled in the art is aware of the fact that the yield of secreted Histidine-tagged pro-carboxypeptidase B obtainable from growth medium, such as liquid growth medium, can be increased when the number of copies of the nucleotide sequence encoding the pre-protein is increased. Thus, the yield of secreted Histidine-tagged pro-carboxypeptidase B molecule obtainable from growth medium can be increased when number of copies of the vector in the genome of the methylotrophic yeast strain is increased. For example, the copy number of the vector can be increased by subjecting the methylotrophic yeast strain to repeated transformations of the vector and repeated selection rounds using increasing concentrations of the selective agent against which the selective marker comprised in the vector confers resistance (US 5,324,639; Thill, G.P., et al., Positive and Negative Effects of Multi-Copy Integrated Expression in Pichia pastoris, International Symposium on the Genentics of Microorganisms 2 (1990), pp. 477-490; Vedvick, T., et al., J. Ind. Microbiol. 7 (1991) 197-201).

The person skilled in the art is also aware of the fact that repeated transformations can be carried out using more than one vector. For example, repeated transformations can be carried out using a first and a second vector, whereby the first and the second vector encode the same pre-protein, whereby in the first and in the second vector the nucleotide sequence encoding the pre-protein is operably linked to a promoter or promoter element, whereby the same pre-protein is expressed and Histidine-tagged pro-carboxypeptidase B is secreted, and whereby the first and the second vector confer resistance to a first and a second selection marker.

An example for a first selective marker is the Sh ble gene, that is the Zeocin™ resistance gene (Drocourt, D., et al., Nucleic Acids Res. 18 (1990) 4009; Carmels, T., et al., Curr. Genet. 20 (1991) 309-314). The protein encoded by the Sh ble gene binds Zeocin™ stoichiometrically and with a strong affinity. The binding of Zeocin™ inhibits its toxic activity thereby selecting for transformants containing the Sh ble gene. It is known to a person skilled in the art that increasing the concentration of Zeocin™ as the selective agent in the medium selects for an increase in the number of copies of the vector expressing the Sh ble gene. It is therefore advantageous to use a vector with the Sh ble gene as a selectable marker to generate by repeated transformation multiple transformants of the methylotrophic yeast strain containing multiple copies of the vector. It is furthermore advantageous that transformations are repeated and selection for even more resistant transformants is repeated until for the transformed methylotrophic yeast strain no further increase of the level of resistance to Zeocin™ is obtained anymore or no further increase of the Zeocin™ concentration in the selection medium is possible anymore.

In case a first and a second vector are used, an example for a second selection marker is resistance against aminoglycoside antibiotics (Southern, P.J., and Berg, P., J. Mol. Appl. Genet. 1 (1982) 327-341) such as G418. Thus, an exemplarily second vector expresses a resistance gene that confers resistance against G418. For example, there are several aminoglycoside phosphotransferases known to the art that confer resistance to aminoglycoside antibiotics (van Treeck, U., et al., Antimicrob Agents Chemother. 19 (1981) 371-380; Beck, E., et al., Gene 19 (1982) 327-336). The aminoglycoside phosphotransferase I (APH-I) enzyme has the ability to inactivate the antibiotic G418 and is an established selectable marker in yeast (Chen, X.J., and Fukuhara, H., Gene, Vol. 69 (1988) 181-192).

Thus, for the purpose of further increasing the dosage of the nucleotide sequence encoding the pre-protein, the second vector is advantageously used for further rounds of transformation and selection, whereby in this case a preferred selective agent is G418 and whereby for transformation the methylotrophic yeast strain transformed with the first vector is used.

It is further preferred the methylotrophic yeast strain is a Hansenula, Pichia, Candida or Torulopsis species. In a very much preferred embodiment of the invention, the methylotrophic yeast strain is selected from the group consisting of Pichia pastoris, Hansenula polymorpha, Candida boidinii and Torulopsis glabrata.

Even more preferred Pichia pastoris strains are deposited at the American Type Culture Collection (ATCC) with the accession numbers 201178, 201949, 204162, 204163, 204164, 204165, 204414, 204415, 204416, 204417, 20864, 28485, 34614, 60372, 66390, 66391, 66392, 66393, 66394, 66395, 76273, 76274, and 90925.

Yet, an even more preferred methylotrophic yeast strain is the Pichia pastoris strain with the American Type Culture Collection accesssion number 76273 or a derivative thereof.

Even more preferred Hansenula polymorpha strains are deposited at the American Type Culture Collection with the accession numbers 14754, 200499, 200500, 200501, 200502, 200503, 200504,200505,200506,200507,200508,200509,200510, 200511, 200512, 200513, 200838, 200839, 201322, 204205, 22023, 26012, 34438, 36669, 38626, 44954, 44955, 46059, 48180, 58401, 62809, 64209, 66057, 76722, 76723, 76760, 90438, 96694, 96695, MYA-335, MYA-336, MYA-337, MYA-338, MYA-339, and MYA-340.

Even more preferred Candida boidinii strains are deposited at the American Type Culture Collection with the accession numbers 18810, 201209, 20432, 26175, 32195, 32929, 36351, 38256, 38257, 44637, 46498, 48180, 56294, 56507, 56897, 60364, 62807, 90439, 90441, 96315, and 96926.

Even more preferred Torulopsis glabrata strains are deposited at the American Type Culture Collection with the accession numbers 15126, 15545, 2001, 22019, 26512, 28226, 28290, 32312, 32554, 32936, 34147, 34449, 36909, 38326, 4135, 46433, 48435, 58561, 66032, 750, and 90030.

Another embodiment of the invention is a transformed Pichia pastoris strain with a chromosome that contains a vector comprising a nucleotide sequence which encodes a pre-protein consisting of the rat pro-carboxypeptidase B that is N-terminally fused to a Histidine-tag and a signal peptide, operably linked with the Pichia pastoris AOX1 promoter according to SEQ ID NO: 5 or a promoter element thereof, whereby the nucleotide sequence that encodes the pre-protein is the nucleotide sequence of SEQ ID NO: 3. It is also preferred that the vector comprises a nucleotide sequence which encodes a pre-protein consisting of the rat pro-carboxypeptidase B that is N-terminally fused to a Histidine-tag (Histidine-tag) and a signal peptide, whereby optionally between the Histidine-tag and the signal peptide a spacer sequence may be inserted.

Yet another embodiment of the invention is a protein with carboxypeptidase B activity which is substantially free of carboxypeptidase B propeptide, obtainable by the method according to the invention. The term "substantially free" denotes that the propeptide is below the detection level using mass spectroscopic detection means. An exemplary analysis is described in Example 8.

As indicated in Example 8, the purified protein with carboxypeptidase B activity predominantly lacks the C-terminal Tyrosine (Tyr497 in SEQ ID NO:4). Only a very small fraction of the purified protein still contains this amino acid. Thus, according to the invention the protein with carboxypeptidase B activity which is substantially free of carboxypeptidase B propeptide has the amino acid sequence of SEQ ID NO:4 from position 191 to position 496.

Example 9 illustrates that after the first purification step according to Example 7 the collected pools contain product with almost equal amounts of product with and without C-terminal Tyrosine. During the process the terminal Tyrosine residues are progressively removed. After the second chromatographic purification step the predominant protein species lacks the C-terminal Tyrosine almost completely. A possible but not tested explanation therefor is that the host organism used for expressing and secreting the product produces a protein with carboxypeptidase Y activity. Carboxypeptidase Y is known from Saccharomyces cerevisiae to be capable of cleaving off C-terminal amino acids with a broad specificity. Although carboxypeptidase Y is known to be a vacuolar enzyme (Kato, M. et al. Eur. J. Biochem 270 (2003) 4587-4593) lysed yeast cells may give rise to noticeable carboxypeptidase Y activity in the fermentation broth. However, since removal of the C-terminal Tyrosine leaves the overall enzymatic properties of the protein with carboxypeptidase B activity unchanged and since no further amino acids are removed from the C-terminus, inactivation or additional separation of the hypothetical carboxypeptidase Y activity is not deemed to be necessary.

Yet another embodiment of the invention is the use of a protein with carboxypeptidase B activity which is substantially free of carboxypeptidase B propeptide according to the invention for proteolytic cleavage of a peptide bond. It is preferred that cleavage of a peptide bond is effected by catalyzing hydrolysis of the basic amino acids, Lysine, Arginine, and Ornithine from the C-teminal position in a polypeptide. It is further preferred that the amino acid sequence of the protein with carboxypeptidase B activity is the amino acid sequence from position 191 to position 496 of SEQ ID NO: 4.

Very much preferred is the use of a protein with carboxypeptidase B activity which is substantially free of carboxypeptidase B propeptide according to the invention characterized in that a peptide bond of a precursor of insulin is cleaved. Processes therefor have been described, e.g., in EP 0 264 250 and EP 0 195 691. Accordingly, a precursor of insulin is proteolytically cleaved by trypsin and a protein with carboxypeptidase B activity. It is even more preferred that the protein with carboxypeptidase B activity catalyzes the hydrolytic cleavage of the basic amino acids, Lysine, Arginine, and Ornithine, from a C-teminal position of the tryptic proteolysis product of the insulin precursor.

Yet another embodiment of the invention is a reagent solution containing a protein with carboxypeptidase B activity which is substantially free of carboxypeptidase B propeptide, according to the invention. It is preferred that the protein with carboxypeptidase B activity in the reagent solution is capable of catalyzing the hydrolytic cleavage of the basic amino acids, Lysine, Arginine, and Ornithine, from a C-teminal position of a peptide or a polypeptide. It is even more preferred that the the amino acid sequence of the protein with carboxypeptidase B activity in the reagent solution is the amino acid sequence from position 191 to position 496 of SEQ ID NO: 4. A reagent solution containing a protein with carboxypeptidase B activity usually is an aequous solution which in addition contains buffer salts. However, further ingredients are possible and are well known to the skilled artisan. An example for a further ingredient is trypsin.

The following examples, references, sequence listings and figures are provided to aid the understanding of the present invention, the true scope of which is set forth in the appended claims. It is understood that modifications can be made in the procedures set forth without departing from the spirit of the invention.

## Description of the Figures

**Figure 1** Graphic representation of the vector pCPB-1. The vector contains a nucleotide sequence encoding a pre-protein, that is a fusion protein comprising the α-factor signal peptide from Saccharomyces cerevisiae (denoted "alpha-Faktor-SS") and the Histidine-tagged pro-carboxypeptidase B. The nucleotide sequence encoding a pre-protein is under transcriptional control of the AOX1 promoter (denoted "AOX1-Promotor") and the AOX1 terminator (denoted "AOX1-TT"). The vector confers resistance against Zeocin®.

**Figure 2a** Diagram depicting the result of an exemplary mass spectrometry analysis of a carboxypeptidase B preparation according to Example 8. The x-axis indicates m/z values (m = ion mass; z = ion charge). The asterisks denote peaks which correspond to the respective carboxypeptidase B dimers.

**Figure 2b** Diagram depicting the result of an exemplary mass spectrometry analysis of a carboxypeptidase B preparation according to Example 8. The x-axis indicates ion molecular weight in [Da].

**Figure 3** Section (a) shows the hypothetical m/z values and peaks which correspond to the carboxypeptidase B propeptide, also referred to as the 12,132 Da peptide. The section denoted (b) shows the corresponding clipping of the spectrum according to Figure 1a.
The x-axes of sections (a) and (b) are aligned and have identical scaling.

**Figure 4** Section (a) shows the hypothetical m/z values and peaks which correspond to the carboxypeptidase B propeptide, also referred to as the 10,746 Da peptide. The section denoted (b) shows the corresponding clipping of the spectrum according to Figure 1a.
The x-axes of sections (a) and (b) are aligned and have identical scaling.

**Figure 5** Clipping of a spectrum obtained with the enzyme preparation according to Example 8.

**Figure 6** Diagram depicting the result of mass spectrometry analysis of a carboxypeptidase B preparation. Analysis was performed on a sample taken after the expanded bed chromatography, as explained in Example 9. The x-axis of section (a) indicates m/z values (m = ion mass; z = ion charge). The x-axis of section (b) indicates ion molecular weight in [Da].

**Figure 7** Diagram depicting the result of mass spectrometry analysis of a carboxypeptidase B preparation. Analysis was performed on a sample taken after the Q-Sepharose ff™, as explained in Example 9. The x-axis of section (a) indicates m/z values (m = ion mass; z = ion charge). The x-axis of section (b) indicates ion molecular weight in [Da].

**Figure 8** Diagram depicting the result of mass spectrometry analysis of a carboxypeptidase B preparation. Analysis was performed on the final product of the purification procedure, as explained in Example 9. The x-axis of section (a) indicates m/z values (m = ion mass; z = ion charge). The x-axis of section (b) indicates ion molecular weight in [Da].

## Example 1

### Synthesis of the gene encoding the pre-protein

DNA techniques were performed according to standard procedures (Sambrook, Fritsch & Maniatis, Molecular Cloning, A Laboratory Manual, 3rd edition, CSHL Press, 2001). Reagents for molecular biological work were used according to the recommendations of the suppliers.

A nucleotide sequence encoding an artificial pre-pro-carboxypeptidase B gene according to SEQ ID NO: 3 was synthesized de-novo. Portions of the nucleotide sequence were synthesized as 24 single-stranded DNA oligonucleotides with a length of between 54 and 90 nucleotides. The single-stranded oligonucleotides represented an alternating fashion overlapping portions of the leading strand and the lagging strand of SEQ ID NO: 3. Each oligonucleotides was designed such that the 5' and 3' ends overlapped with the neighbouring oligonucleotide. As an exception, the oligonucleotides representing the 5' and the 3' terminus of SEQ ID NO: 3 only overlapped with the 3' and 5' ends of the neighbouring fragments, respectively. The sequences of the overlaps were chosen such that during an annealing reaction unspecific annealing was avoided. The oligonucleotides representing the 5' and the 3' terminus of SEQ ID NO: 3 additionally contained linker sequences with recognition sites for restriction endonucleases, in order to facilitate further steps of molecular cloning, such as the insertion of the artificial coding sequence into expression vectors. A preferred restriction site for the oligonucleotides representing the 5' terminus of SEQ ID NO: 3 was XhoI. A preferred restriction site for the oligonucleotides representing the 3' terminus of SEQ ID NO: 3 was NotI.

A nucleic acid molecule comprising the nucleotide sequence according SEQ ID NO: 3 was synthesized stepwise by way of PCR (polymerase chain reaction). In principle, the first two oligonucleotides representing adjacent and partially overlapping portions of the leading and the lagging strand were added to a PCR reaction mixture. Following several cycles of PCR, a contiguous double-stranded fragment representing both portions of the leading and the lagging strand was obtained. The double-stranded fragment was optinally purified, mixed with the consecutive adjacent and partially overlapping single-stranded oligonucleotide and was subjected to a further round of PCR.

Using the procedure described above, three larger fragments of the nucleotide sequence according SEQ ID NO: 3 were synthesized independently. Each fragment could be present in a mixture of by-products. Therefore, the fragments were electrophoresed in an agarose gel and identified by their size. Agarose blocks containing the desired fragments were excised and the DNA fragments were isolated using the QiaQuick Gel Extraction Kit (Qiagen). Other extraction methods are also possible.

Of the three fragments, the second fragment at its 5' end had a sequence overlap with the 3' end of the first fragment, and whereby the second fragment at its 3' end had a sequence overlap with the 5' end of the third fragment. The full-length sequence according to SEQ ID NO: 3 was again synthesized in a stepwise fashion. The three fragments were united in a PCR reaction mixture. The annealing temperatures were chosen taking into account the overlapping sequence with the lowest melting point. Five cycles of PCR were performed, followed by the addition of an additional pair of primers complementary to the 5' end and the 3' end of the desired full-length product. Using an annealing temperature chosen with respect to the annealing temperature of the newly added primer with the lower melting temperature, further 25 cycles of PCR were performed and the full-length fragment comprising the nucleotide sequence of SEQ ID NO: 3 was obtained. The full-length fragment (i.e. the full-length pre-protein-encoding DNA including linker sequences with restriction endonuclease cleavage sites) was inserted into a vector and propagated in a transformed host organism. A preferred host organism for propagation purposes was E. coli.

The nucleotide sequence of the full-length DNA comprising the sequence encoding the pre-protein of SEQ ID NO: 4 was verified by sequencing. The final full-length product was amplified using PCR.

## Example 2

### Construction of vectors, transformation, expression

For further steps regarding the construction of expression vectors, transformation, expression of the pre-protein and secretion of Histidine-tagged pro-carboxypeptidase B into growth media, the methods suggested and described in the Invitrogen manuals "Pichia Expression Kit" Version M 011102 25-0043, "pPICZ A, B, and C" Version D 110801 25-0148, "pPICZ$\alpha$ A, B, and C" Version E 010302 25-0150, and "pPIC9K" Version E 030402 25-0106 were applied. Reference is also made to further vectors, yeast strains and media mentioned therein. Basic methods of molecular biology were applied as described in Sambrook, Fritsch & Maniatis, Molecular Cloning, A Laboratory Manual, 3rd edition, CSHL Press, 2001.

As a result, several vectors were obtained whereby each comprised an expression cassette containing capable of expressing the pre-protein according to SEQ ID NO: 4 in methylotrophic yeast strains. A preferred yeast strain was a Pichia pastoris strain. Each vector in addition comprised, among other genetic elements, an origin of replication capable of replicating the vector autonomously in methylotrophic yeast cells. In addition the vectors were designed such as to enable integration into the genome of the host cell. Another genetic element on each vector was a further expression cassette expressing a selectable marker.

Repeated transformations of the Pichia pastoris host strain were performed in order to increase the yield of secreted Histidine-tagged pro-carboxypeptidase B.

**Example 3**

**Protein quantification**

Quantification of purified rat carboxypeptidase B was performed by way of measuring the extinction at 280 nm using cuvettes having a width of 1 cm. The concentration was determined according to the following formula:

$$\text{Protein [mg/ml]} = \frac{10\ \text{[mg/ml]} * \Delta E_{\text{Probe}} * \text{dilution factor}}{21.4}$$

**Example 4**

**Protein activity**

The activity of carboxypeptidase B was determined using 1.5 M Hippuryl-L-Arginine (Bachem G-2265) in 100 mM Tris pH 7.8 at 25°C and cuvettes having a width of 0.5 cm.

**Example 5**

**HPLC analytics**

Activation of carboxypeptidase B from Histidine-tagged pro-carboxypeptidase B was monitored using HPLC (high pressure liquid chromatography) and gel filtration separation means (Superdex 75 HR 10/30, Amersham Biosciences). The chromatography buffer was 0.1 M Tris pH 7.5, 0.3 M NaCl. The flux was 0.5 ml/min.

Retention times of carboxypeptidase B and Histidine-tagged pro-carboxypeptidase B differed unambiguously (1.2 ml) facilitating their recognition on elution profiles.

**Example 6**

**Fermentation**

General methods were followed according to descriptions in the literature (Higgins, D.R., Cregg, J.M. (1998) Pichia Protocols. In: Methods in Molecular Biology Vol. 103, the whole document but in particular pp 107-120; Stratton, J., Chiruvolu, V., Meagher, M. (1998) High cell-density fermentation. In: Pichia Fermentation Guidelines, Invitrogen).

Pre-cultures of the transformed Pichia pastoris strain capable of expressing the pre-protein of SEQ ID NO: 4 were made at 30°C in yeast nitrogen base medium without amino acids (DIFCO). The pre-culture was used to inoculate a fermentation medium. The fermentation medium contained minerals, that is to say $H_3PO_4$, $CaSO_4$ x 2 $H_2O$, $K_2SO_4$, $MgSO_4$ x 7 $H_2O$, KOH, NaOH, the trace elements $CuSO_4$ x 5 $H_2O$, KJ, $MnSO_4$ x $H_2O$, $Na_2MoO_4$ x 2 $H_2O$, $H_3BO_4$, $ZnCl_2$, $CoCl_2$ x 6 $H_2O$, and $FeSO_4$ x 7 $H_2O$. The fermentation medium also contained biotin and glycerol. The pH was adjusted to pH 5.0 and maintained at that value using $NH_3$ which at the same time served as a source for Nitrogen.

When glycerol being the first carbon source was exhausted, methanol was added, thereby inducing expression of the pre-protein that was driven by the AOX1 promoter.

**Example 7**

**Purification**

The fraction of the liquid mass of the fermentation broth was adjusted to 5-13% by adding a buffer containing 20 mM Tris pH 7.5, 1M NaCl and 1 mM imidazole. The diluted fermentation broth was passed through a column with a particulate metal chelate affinity matrix. The preferred particulate metal chelate affinity matrix was $Zn^{2+}$-loaded StreamlineTM Chelating (Amersham Biosciences). Loading the particulate metal chelate affinity matrix in the column with Histidine-tagged pro-carboxypeptidase B was done using the expanded bed chromatography technique. Under the conditions as described above, the Histidine-tagged pro-carboxypeptidase B in the diluted fermentation broth binds, i.e. adsorbs to the particulate metal chelate affinity matrix.

Following the binding step, the particulate metal chelate affinity matrix in the (still expanded) column was washed with a washing buffer containing 20 mM Tris pH 7.5, 1 M NaCl, and 1 mM Imidazole. Several (at least two) washing steps were performed under conditions allowing Histidine-tagged pro-carboxypeptidase B to remain bound to the particulate metal chelate affinity matrix . The first washing step was performed using the washing buffer additionally containing 11% [volume by volume] of glycerol. Any additional washing step was performed using the washing buffer without glycerol.

Activation of carboxypeptidase B was effected by way of on-column cleavage with trypsin. Following the washing step, a buffer (20 mM Tris pH 7.5, 1M NaCl and 1 mM imidazole) containing trypsin (13-20 U/ml) was pumped through the column, whereby the buffer that had left the column was re-cycled into the column. After 150-300 min the buffer now additionally containing activated carboxypeptidase B was removed. The column was washed (two to three washing steps) in upward flow mode using washing buffer, whereby the flow-through was collected. The buffer now additionally containing activated carboxypeptidase B and the flow-through from the washing steps were pooled. Tryptic activity was stopped by adding Benzamidinium hydrochloride to a final concentration of 1 mM.

The buffer containing activated carboxypeptidase B and trypsin was filtered to remove last traces of residual biomass. The filtered buffer was subjected to diafiltration by tangential flow filtration. A dialysis buffer was used which contained Tris pH 8.3 at a concentration of 60-100 mM, and further containing 1 mM Benzamidinium hydrochloride, 0.1 mM $ZnCl_2$. Diafiltration resulted in an exchange of the buffer in which the activated carboxypeptidase was present.

The subsequent step was Q-Sepharose ff™ chromatography. The buffer in which the activated carboxypeptidase was present was loaded on a column containing Q-Sepharose ff™. A washing step was applied using 60-100 mM Tris pH 8.3, 1 mM Benzamidinium hydrochloride, 0.1 mM $ZnCl_2$. Following the washing step, the column was eluted by applying a gradient extending to 125 mM NaCl. Fractions were collected. Fractions containing carboxypeptidase B were pooled.

## Example 8

### Mass spectrometry of purified carboxypeptidase B

Carboxypeptidase B was purified according to Example 7. Prior to mass spectrometry the enzyme preparation was desalted using a micro HPLC device (ABI-120A) equipped with an autosampler (Gilson 234). The column used was a Vydac Protein C4 cartridge (Vydac catalogue no. 214GD51) together with the appropriate guard holder. An aliquot of the enzyme preparation containing 50 μg - 100 μg of protein was loaded on the column and eluted using a gradient of elution buffer A (3.5% "pro analysi" grade HCOOH [formic acid] in HPLC grade water [Baker]) and buffer B (80% HPLC grade acetonitrile, 5% HPLC grade HCOOH in HPLC grade water [Baker]). The gradient was applied by eluting for the first 5 min with 5% buffer B, 95% Buffer A and then for 3 more min with 100% buffer B. During HPLC the temperature of the column was kept at 35°C. The enzyme was detected at a wave length of 280 nm. It was found that the enzyme eluted between $t_{5\ min}$ and $t_{8\ min}$. The protein peak was collected (from 30 mAU - 40 mAU; peak maximum >800 mAU).

Mass spectrometry was performed on a Q-Tof 2™ (Waters Micromass, Manchester, UK) equipped with a nanospray interface. The device was capable of selecting peptide or protein ions with an m/z of 200 - 2000 and protein ions with an m/z of 800 - 2000. Spray capillaries were from Proxeon ("Medium", catalogue no. ES 387).
Measurements were taken using variable parameters with respect to capillary voltage, cone voltage, and MS profiles. Parameters were adjusted for each sample to be analyzed regarding the measurement range. For analysis the software MassLynx 4.0™ with the MaxEnt 1™ package (Waters) was used. Using Maximum Entropy processing applied to mass spectrometry data MaxEnt 1™ aids the enhancement of complex spectra by allowing the deconvolution of overlapping multiply-charged spectra produced by the analysis of protein mixtures. Indicated masses below 10,000 Da are monoisotopic, masses indicated as equal to or above 10,000 Da are averaged masses.

By way of mass spectrometry it was found that the enzyme preparation, i.e. the purified carboxypeptidase B gives rise to essentially three different mass peaks: (1) the main peak [designated A in Figure 2b] corresponding to a (averaged) mass of 35,015 Da indicating carboxypeptidase B lacking the C-terminal Tyrosine; (2) a minor peak corresponding to a mass of about 70,026 Da was also found (not shown) and indicates the dimer of carboxypeptidase B lacking the C-terminal Tyrosine; (3) a minor peak corresponding to a mass of about 35,176 Da indicates the carboxypeptidase B including the C-terminal Tyrosine.

Theoretically, peaks corresponding to the Histidine-tagged propeptide should reflect the 12,132 Da peptide of 105 amino acids generated from the amino acid sequence of of SEQ ID NO: 4 by (a) the KEX-2 signal peptidase cleaving between Arg85 and Ser86 and (b) trypsin cleaving between Arg190 and Ala191. Also, fragments of the Histidine-tagged propeptide can be postulated. These include the 10,746 Da peptide comprising the sequence from Ser86 to Arg178 and the 1,404 Da peptide comprising the sequence from Asn179 to Arg190. The "(a)" sections of Figures 3 and 4 indicate the hypothetical peaks that one would expect if the 12,132 Da and 10,746 Da peptides were present in the enzyme preparation. The "(b)" sections represent enlarged clippings of spectra like the one shown in Figure 2a. It can be seen that at the positions of the hypothetical peaks nothing is detected in the spectra of the enzyme preparation. In addition, Figure 5 shows a clipping of a spectrum obtained with the enzyme preparation. The hypothetical 1,404 Da peptide would be expected to give gise to peaks at m/z = 702.8 ([M+2H]2+) and m/z = 468.9 ([M+3H]3+). However, no conspicuous peaks indicating the presence of the propeptide are present.

Furthermore, the N-terminal amino acid sequence of the polypeptide corresponding to the main peak was determined. The 15 N-terminal amino acids were found to be "ASGHSYTKYNNWETI". These are identical with the amino acids 191 to 205 of SEQ ID NO: 4, i.e. they represent the N-terminus of the activated carboxypeptidase B enzyme.

## Example 9

### Removal of C-terminal Tyrosine

Carboxypeptidase B was purified according to Example 7. A sample was taken from the pooled carboxypeptidase B containing fractions and washing solutions. Mass spectrometry was performed according to Example 8. Results are shown in Figure 6. Figure 7 illustrates the results of a sample after the Q-sepharose ff™ chromatography and Figure 8 illustrates the endproduct. It becomes clear that in the beginning about 50% or more of carboxypeptidase B contains the C-terminal Tyrosine. After the second chromatography the predominant protein species lacks the C-terminal Tyrosine already. However, C-terminal degradation was restricted to the C-terminal Tyrosine.

## List of References

Alberts, B., Johnson, A., Lewis, J., Raff, M., Roberts, K., Walter, P. (eds), Molecular Biology of the Cell, fourth edition, 2002, Garland Science Publishing

Beck, E., et al., Gene 19 (1982) 327-336

Borsing, L. et al. Biochem. Biophys. Res. Commun. 240 (1997) 586-590

Carmels, T., et al., Curr. Genet. 20 (1991) 309-314

Chaga, G. et al., Biotechnol. Appl. Biochem. 29 (1999) 19-24

Chaga, G. et al., J. Chromatogr. A 864 (1999) 257-256

Chen, B.P. and Hai, T., Gene 139(1994) 73-75

Chen, X.J., and Fukuhara, H., Gene, Vol. 69 (1988) 181-192

Clauser E. et al., J. Biol. Chem. 1988, Vol. 263, 17837 - 45

DE 19 915 938

Drocourt, D., et al., Nucleic Acids Res. 18 (1990) 4009

EP 0 116 201

EP 0195 691

EP 0 253 303

EP 0 264 250

EP 0 282 042

EP 1 069 131

Folk J. Carboxypeptidase B, in: The Enzymes 3, P. Boyer, Academic Press, NY, 57, 1971

Folk, J., and Gladner, J.: Biochim Biophys Acta (1961) 48, 139-47

Hakansson, K. et al. Acta Crystallogr. D Biol. Crystallogr. 56 (2000) 924-926

Halliwell, C.M. et al., Anal Biochem. 295 (2001) 257-261

Higgins, D.R., Cregg, J.M. (1998) Pichia Protocols. In: Methods in Molecular Biology Vol. 103, the whole document but in particular pp 107-120

Hochuli, E. et al., Bio/Technology 6 (1988) 1321-1325

Hochuli, E. et al., J. Chromatogr. 411 (1987) 177-184

Janknecht, R. and Nordheim, A., Gene 121 (1992) 321-324

Janknecht, R. et al. Proc. Natl. Acad. Sci. USA 88 (1991) 8972-8976

Julius, D., et al., Cell 37 (1984) 1075-1089

Kaslow, D.C. and Shiloach, J., Bio/Technology 12 (1994) 494-499

Kato, M. et al. Eur. J. Biochem 270 (2003) 4587-4593

Kuusinen, A. et al., Eur. J. Biochem. 233 (1995) 720-726

Pharmacia Biotech manual "Expanded bed adsorption, principles and methods". ISBN 91-630-5519-8

Porath, J. et al., Nature 258 (1975) 598-599

Rank, K.B. et al., Protein Expr. Purif. 22 (2001) 258-266

Sambrook, Fritsch & Maniatis, Molecular Cloning, A Laboratory Manual, 3rd edition, CSHL Press, 2001

Schmidt, M. et al. Protein Expr. Purif. 12 (1998) 323-330
Southern, P.J., and Berg, P., J. Mol. Appl. Genet. 1 (1982) 327-341
Stratton, J., Chiruvolu, V., Meagher, M. (1998) High cell-density fermentation. In: Pichia Fermentation Guidelines, Invitrogen
Thill, G.P., et al., Positive and Negative Effects of Multi-Copy Integrated Expression in Pichia pastoris, International Symposium on the Genentics of Microorganisms 2 (1990), pp. 477-490
US 4,683,293
US 4,808,537
US 4,812,405
US 4,818,700
US 4,837,148
US 4,855,231
US 4,857,467
US 4,870,008
US 4,879,231
US 4,882,279
US 4,885,242
US 4,895,800
US 4,929,555
US 5,002,876
US 5,004,688
US 5,032,516
US 5,122,465
US 5,135,868
US 5,166,329
US 5,284,933
US 5,310,663
US 5,324,639
US 5,618,676
US 5,854,018
US 5,856,123
US 5,919,651
van Treeck, U., et al., Antimicrob Agents Chemother. 19 (1981) 371-380 Vedvick, T., et al., J. Ind. Microbiol. 7 (1991) 197-201
Ventura et al. J. Biol. Chem. (1999) 274(28), 19925-33
Waters et al., J. Biol. Chem. 263 (1988) 6209-14
Werten, M.W., et al., Yeast 15 (1999) 1087-1096
WO 00/56903
WO 01/51624
WO 96/23064
Wu, J. and Filutowicz, M., Acta Biochim. Pol. 46 (1999) 591-599
Zisapel, N. and Sokolovsky, M, Eur J Biochem (1975) 54, 541-7

## Claims

1. A method to produce a protein with carboxypeptidase B activity, comprising the steps of

(a) providing a vector comprising a nucleotide sequence which encodes a pre-protein consisting of the rat pro-carboxypeptidase B that is N-terminally fused to a Histidine-tag and a signal peptide,
whereby the signal peptide is the N-terminal sequence of the pre-protein,
and optionally between the Histidine-tag and the signal peptide or between the Histidine-tag and rat carboxypeptidase B a spacer sequence is inserted;
(b) transforming a microbial host organism with the vector;
(c) cultivating the microbial host organism in a growth medium containing nutrients and a carbon source, whereby the microbial host organism expresses the pre-protein and secretes the Histidine-tagged pro-carboxypeptidase B into the growth medium;
(d) immobilizing the secreted Histidine-tagged pro-carboxypeptidase B in the growth medium of step (c) on a particulate metal chelate affinity matrix capable of binding the Histidine-tag, and washing the particulate metal chelate affinity matrix, whereby the Histidine-tagged pro-carboxypeptidase B is immobilized;
(e) incubating the particulate metal chelate affinity matrix with the immobilized Histidine-tagged pro-carboxypeptidase B of step (d) in a buffer containing trypsin, thereby cleaving proteolytically the pro-carboxypeptidase B moiety and releasing the protein with carboxypeptidase B activity into the liquid phase,
whereby the Histidine-tagged propeptide moiety is immobilized;
(f) separating the liquid phase containing the protein with carboxypeptidase B activity from the particulate metal chelate affinity matrix,
whereby the Histidine-tagged propeptide moiety is immobilized; and
(g) purifying the protein with carboxypeptidase B activity from the liquid phase of step (f).

2. The method according to claim 1, **characterized in that** the microbial host strain is a methylotrophic yeast strain.

3. The method according to any of the claims 1 or 2, **characterized in that** the amino acid sequence of the rat pro-carboxypeptidase B is the amino acid sequence from position 14 to position 415 in SEQ ID NO: 3.

4. The method according to any of the claims 1 to 3, **characterized in that** the signal peptide contains a signal peptidase cleavage site which is located adjacent to the Histidine-tag or adjacent to the spacer sequence.

5. The method according to any of the claims 1 to 4, **characterized in that** the amino acid sequence of the expressed pre-protein is the amino acid sequence in SEQ ID NO: 4.

6. The method according to any of the claims 1 to 5, **characterized in that** the nucleotide sequence encoding rat pro-carboxypeptidase B is the nucleotide sequence from position 286 to position 1,497 in SEQ ID NO: 3.

7. The method according to any of the claims 1 to 6, **characterized in that** the nucleotide sequence encoding the pre-protein is the nucleotide sequence in SEQ ID NO: 3.

**Patentansprüche**

1. Verfahren zur Herstellung eines Proteins mit Carboxypeptidase-B-Aktivität, wobei man in den Verfahrensschritten

(a) einen Vektor bereitstellt, der eine für ein Präprotein, das aus N-terminal mit einem Histidin-Tag und einem Signalpeptid fusionierter Pro-Carboxypeptidase B aus Ratte besteht, codierende Nukleotidsequenz umfasst, womit das Signalpeptid die N-terminale Sequenz des Präproteins ist,
und gegebenenfalls zwischen dem Histidin-Tag und dem Signalpeptid oder zwischen dem Histidin-Tag und Carboxypeptidase B aus Ratte eine Spacer-Sequenz eingefügt wird;
(b) einen mikrobiellen Wirtsorganismus mit dem Vektor transformiert;
(c) den mikrobiellen Wirtsorganismus in einem Nährstoffe und eine Kohlenstoffquelle enthaltendem Wachstumsmedium kultiviert, womit der mikrobielle Wirtsorganismus das Präprotein exprimiert und die mit dem Histidin-Tag versehene Pro-Carboxypeptidase B in das Wachstumsmedium sezerniert;
(d) die sezernierte, mit dem Histidin-Tag versehene Pro-Carboxypeptidase B im Wachstumsmedium aus Schritt (c) auf einer zur Bindung des Histidin-Tag fähigen partikulären Metallchelat-Affinitätsmatrix immobilisiert und die partikuläre Metallchelat-Affinitätsmatrix wäscht, womit die mit dem Histidin-Tag versehene Pro-Carboxypeptidase B immobilisiert wird;
(e) die partikuläre Metallchelat-Affinitätsmatrix mit der immobilisierten, mit dem Histidin-Tag versehenen Pro-Carboxypeptidase B aus Schritt (d) in einem trypsinhaltigen Puffer inkubiert, wodurch die Pro-Carboxypeptidase-B-Gruppierung proteolytisch gespalten und das Protein mit Carboxypeptidase-B-Aktivität in die flüssige Phase freigesetzt wird,
womit die mit dem Histidin-Tag versehene Propeptid-Gruppierung immobilisiert wird;
(f) die das Protein mit Carboxypeptidase-B-Aktivität enthaltende flüssige Phase von der partikulären Metallchelat-Affinitätsmatrix trennt,
womit die mit dem Histidin-Tag versehene Propeptid-Gruppierung immobilisiert wird; und
(g) das Protein mit Carboxypeptidase-B-Aktivität aus der flüssigen Phase aus Schritt (f) aufreinigt.

2. Verfahren nach Anspruch 1, **dadurch gekennzeichnet, dass** es sich bei dem mikrobiellen Wirtsstamm um einen methylotrophen Hefestamm handelt.

3. Verfahren nach einem der Ansprüche 1 oder 2, **dadurch gekennzeichnet, dass** es sich bei der Aminosäuresequenz der Pro-Carboxypeptidase B aus Ratte um die Aminosäuresequenz von Position 14 bis Position 415 in SEQ ID NO: 3 handelt.

4. Verfahren nach einem der Ansprüche 1 bis 3, **dadurch gekennzeichnet, dass** das Signalpeptid eine Signalpeptidase-Spaltstelle enthält, die unmittelbar neben dem Histidin-Tag oder unmittelbar neben der Spacer-Sequenz liegt.

5. Verfahren nach einem der Ansprüche 1 bis 4, **dadurch gekennzeichnet, dass** es sich bei der Aminosäuresequenz des exprimierten Präproteins um die Aminosäuresequenz in SEQ ID NO: 4 handelt.

6. Verfahren nach einem der Ansprüche 1 bis 5, **dadurch gekennzeichnet, dass** es sich bei der für Pro-Carboxypeptidase B aus Ratte codierenden Nukleotidsequenz um die Nukleotidsequenz von Position 286 bis Position 1497 in SEQ ID NO: 3 handelt.

7. Verfahren nach einem der Ansprüche 1 bis 6, **dadurch gekennzeichnet, dass** es sich bei der für das Präprotein codierenden Nukleotidsequenz um die Nukleotidsequenz in SEQ ID NO: 3 handelt.

**Revendications**

1. Procédé d'une production d'une protéine possédant une activité de carboxypeptidase B, comprenant les étapes

   (a) de procuration d'un vecteur comprenant une séquence nucléotidique qui encode une pré-protéine constituée de la pro-carboxypeptidase B du rat qui est fusionnée à son extrémité amino terminale à une étiquette d'histidine et un peptide signal,
   le peptide signal représentant la séquence de l'extrémité amino terminale de la pré-protéine,
   et, de manière facultative, une séquence espaceur étant insérée entre l'étiquette d'histidine et le peptide signal ou bien entre l'étiquette d'histidine et la carboxypeptidase B du rat ;
   (b) de transformation d'un organisme hôte microbien avec le vecteur ;
   (c) de culture de l'organisme hôte microbien dans un milieu de croissance contenant des nutriments et une source de carbone, l'organisme hôte microbien exprimant la pré-protéine et sécrétant la pro-carboxypeptidase B munie d'une étiquette d'histidine dans le milieu de croissance ;
   (d) d'immobilisation de la pro-carboxypeptidase B sécrétée munie d'une étiquette d'histidine dans le milieu de croissance de l'étape (c) sur une matrice d'affinité pour les chélates métalliques, de type particulaire, capable de se lier à l'étiquette d'histidine, et de lavage de la matrice d'affinité pour les chélates métalliques, de type particulaire, donnant lieu à une immobilisation de la pro-carboxypeptidase B munie d'une étiquette d'histidine ;
   (e) d'incubation de la matrice d'affinité pour les chélates métalliques, de type particulaire avec la pro-carboxypeptidase B immobilisée munie d'une étiquette d'histidine de l'étape (d) dans un tampon contenant de la trypsine, pour ainsi cliver par voie protéolytique la fraction de pro-carboxypeptidase B et libérer dans la phase liquide la protéine possédant une activité de carboxypeptidase B
   donnant lieu à une immobilisation de la fraction pro-peptidique munie d'une étiquette d'histidine ;
   (f) de séparation de la phase liquide contenant la protéine possédant une activité de carboxypeptidase B par rapport à la matrice d'affinité pour des chélates métalliques, de type particulaire ;
   donnant lieu à une immobilisation de la fraction pro-peptidique munie d'une étiquette d'histidine ; et
   (g) de purification de la protéine possédant une activité de carboxypeptidase B par rapport à la phase liquide de l'étape (f).

2. Procédé selon la revendication 1, **caractérisé en ce que** la souche de l'hôte microbien est une souche de levure méthylotrophique.

3. Procédé selon la revendication 1 ou 2, **caractérisé en ce que** la séquence d'acides aminés de la pro-carboxypeptidase B du rat est la séquence d'acides aminés allant de la position 14 à la position 415 dans la SEQ ID NO : 3.

4. Procédé selon l'une quelconque des revendications 1 à 3, **caractérisé en ce que** le peptide signal contient un site de clivage de la peptidase signal qui est situé en position adjacente à l'étiquette d'histidine ou en position adjacente à la séquence espaceur.

5. Procédé selon l'une quelconque des revendications 1 à 4, **caractérisé en ce que** la séquence d'acides aminés de la pré-protéine exprimée est la séquence d'acides aminés dans SEQ ID NO : 4.

6. Procédé selon l'une quelconque des revendications 1 à 5, **caractérisé en ce que** la séquence nucléotidique encodant la pro-carboxypeptidase B du rat est la séquence nucléotidique allant de la position 286 à la position 1497 dans la SEQ ID NO :3.

7. Procédé selon l'une quelconque des revendications 1 à 6, **caractérisé en ce que** la séquence nucléotidique encodant la pré-protéine est la séquence nucléotidique dans la SEQ ID NO : 3.

# Figure 1

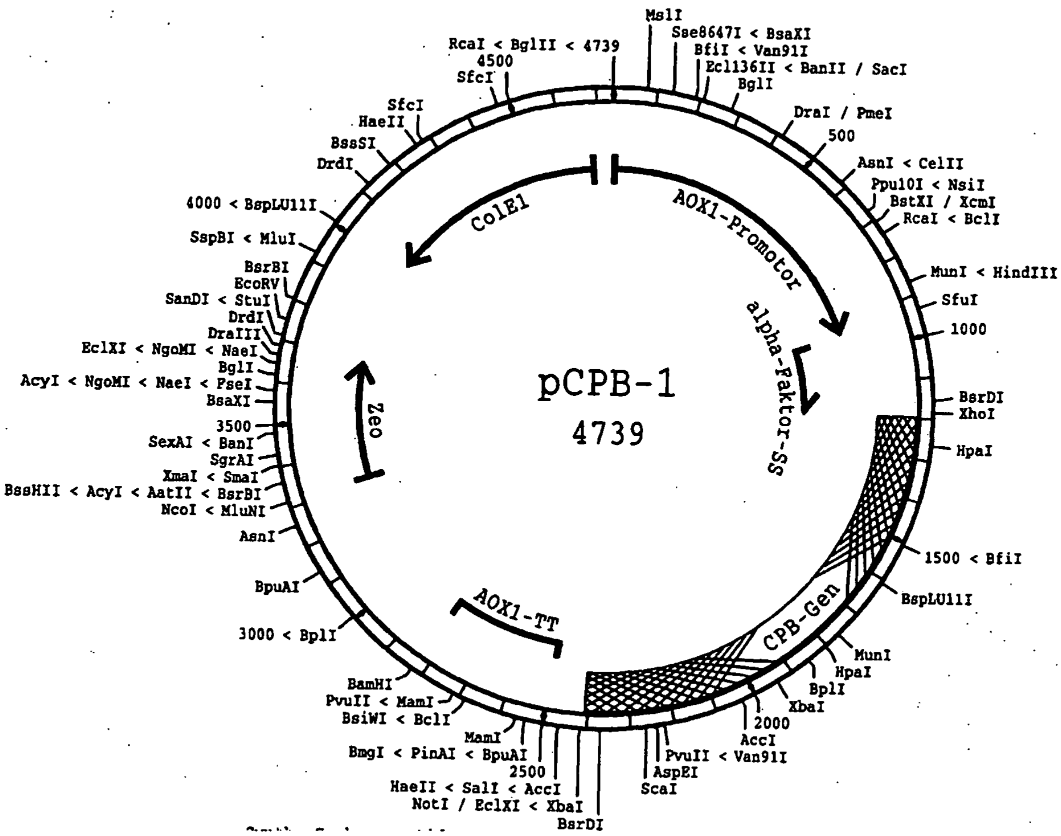
pCPB-1
4739
ColE1
AOX1-Promotor
alpha-Faktor-SS
CPB-Gen
AOX1-TT
Zeo
MslI
Sse8647I < BsaXI
BfiI < Van91I
Ecl136II < BanII / SacI
BglI
DraI / PmeI
500
AsnI < CelII
Ppu10I < NsiI
BstXI / XcmI
RcaI < BclI
MunI < HindIII
SfuI
1000
BsrDI
XhoI
HpaI
1500 < BfiI
BspLU11I
MunI
HpaI
BplI
XbaI
2000
AccI
PvuII < Van91I
AspEI
ScaI
BsrDI
NotI / EclXI < XbaI
HaeII < SalI < AccI
2500
BmgI < PinAI < BpuAI
MamI
BsiWI < BclI
PvuII < MamI
BamHI
3000 < BplI
BpuAI
AsnI
NcoI < MluNI
BssHII < AcyI < AatII < BsrBI
XmaI < SmaI
SgrAI
SexAI < BanI
3500
BsaXI
AcyI < NgoMI < NaeI < PseI
BglI
EclXI < NgoMI < NaeI
DraIII
DrdI
SanDI < StuI
EcoRV
BsrBI
SspBI < MluI
4000 < BspLU11I
DrdI
BssSI
HaeII
SfcI
SfcI
4500
RcaI < BglII < 4739

# Figure 2a

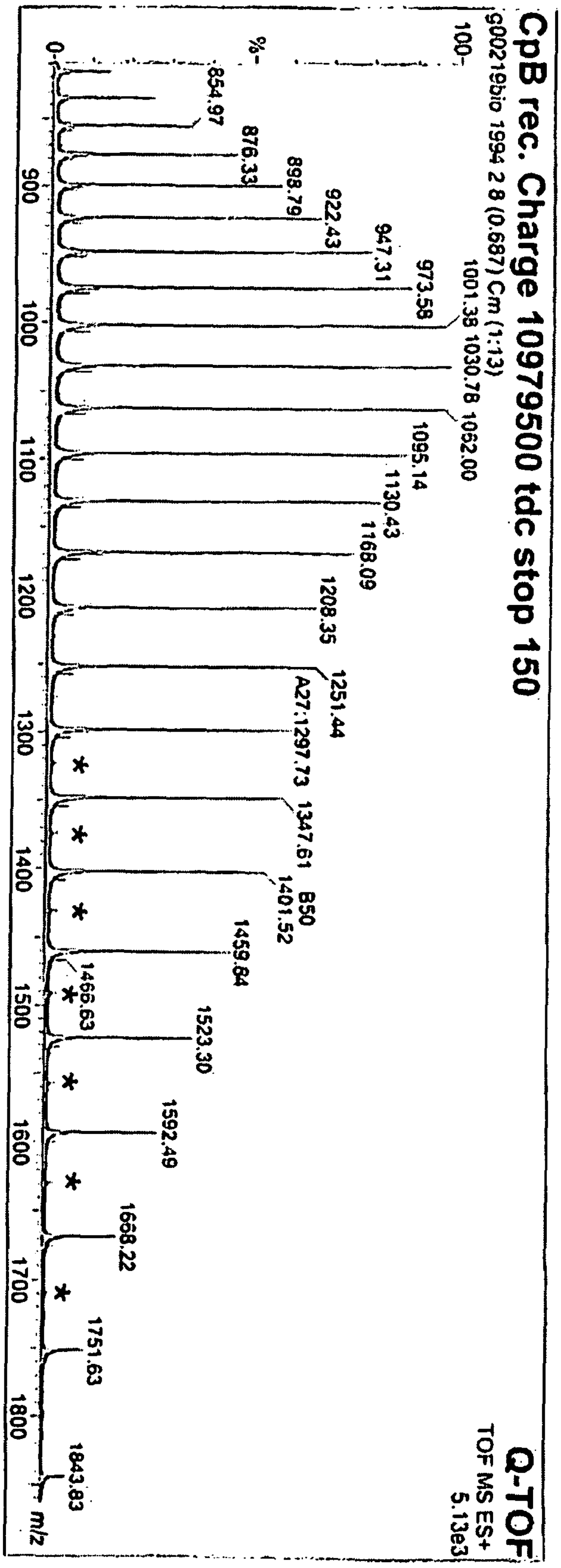

CpB rec. Charge 10979500 tdc stop 150
g0219bio 1994 2 8 (0.687) Cm (1:13)
Q-TOF
TOF MS ES+
5.13e3
100
%
0
854.97
876.33
898.79
922.43
947.31
973.58
1001.38
1030.78
1062.00
1095.14
1130.43
1168.09
1208.35
1251.44
A27:1297.73
1347.61
B50
1401.52
1459.64
1466.63
1523.30
1592.49
1668.22
1751.63
1843.83
900
1000
1100
1200
1300
1400
1500
1600
1700
1800
m/z

# Figure 2b

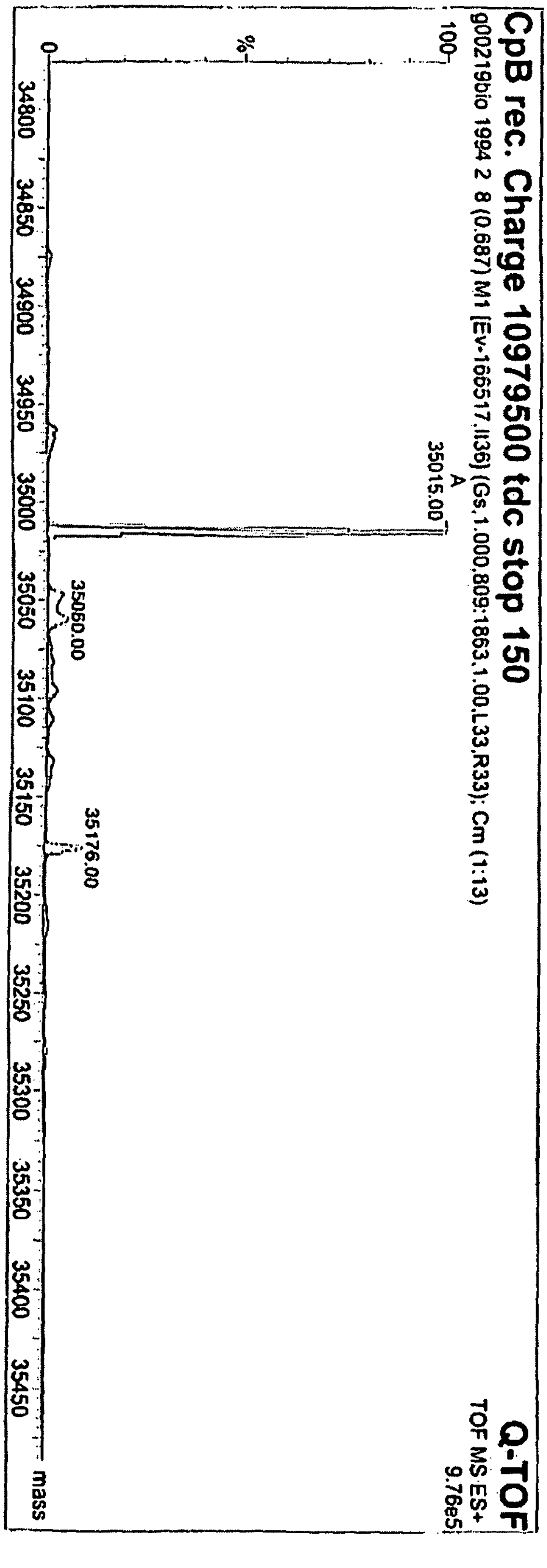
CpB rec. Charge 10979500 tdc stop 150
g0021 9bio 1994 2 8 (0.687) M1 [Ev-166517,It36] (Gs,1.000,809:1863,1.00,L33,R33); Cm (1:13)
Q-TOF
TOF MS ES+
9.76e5
100
%
0
A
35015.00
35060.00
35176.00
34800
34850
34900
34950
35000
35050
35100
35150
35200
35250
35300
35350
35400
35450
mass

Figure 3

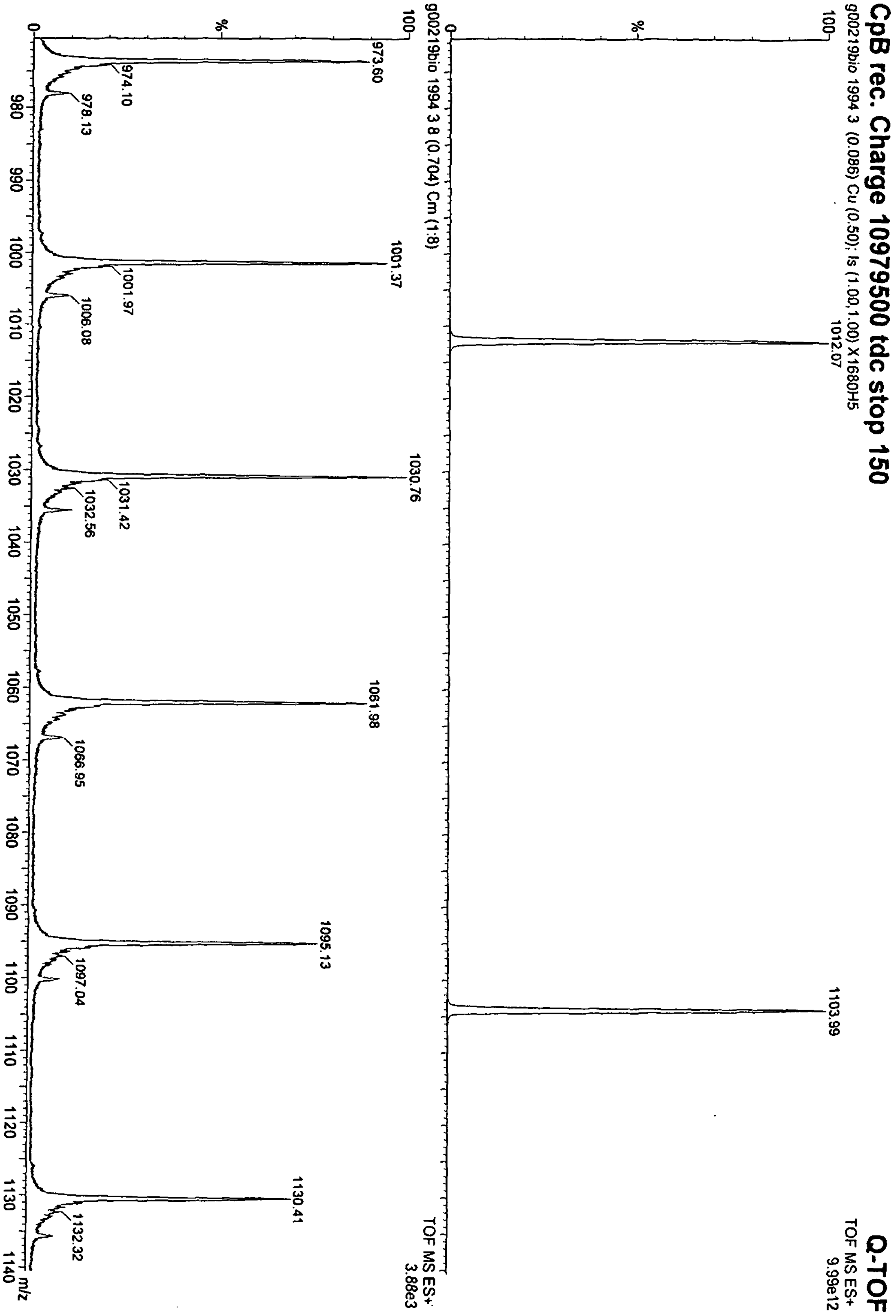
CpB rec. Charge 10979500 tdc stop 150
Q-TOF
(a)
g00219bio 1994 3 (0.086) Cu (0.50); Is (1.00,1.00) X1680H5
TOF MS ES+
9.99e12
100
%
0
1012.07
1103.99
(b)
g00219bio 1994 3 8 (0.704) Cm (1:8)
TOF MS ES+
3.86e3
100
%
0
973.60
974.10
978.13
1001.37
1001.97
1006.08
1030.76
1031.42
1032.56
1061.98
1066.95
1095.13
1097.04
1130.41
1132.32
980
990
1000
1010
1020
1030
1040
1050
1060
1070
1080
1090
1100
1110
1120
1130
1140
m/z

Figure 4

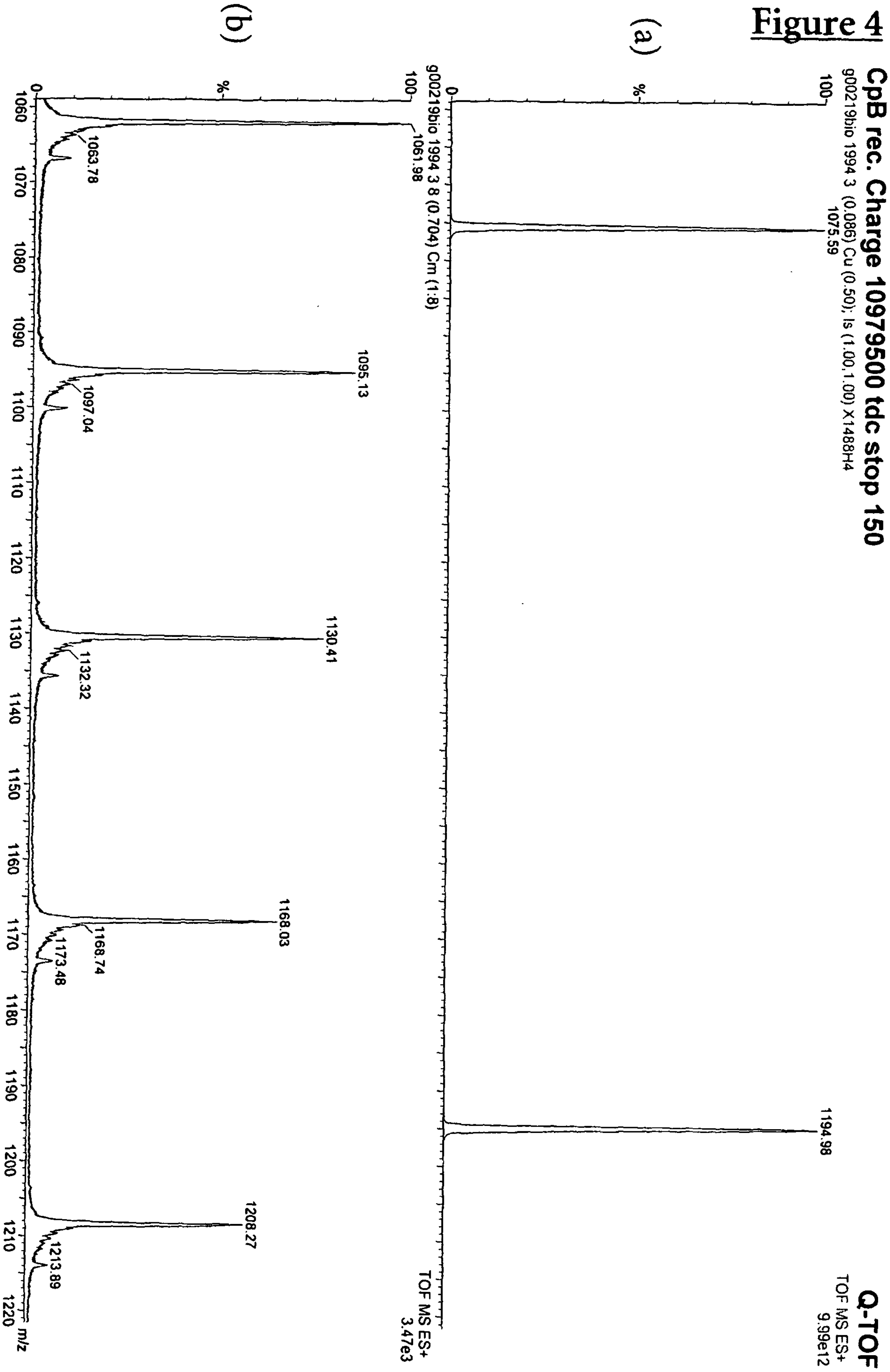
CpB rec. Charge 10979500 tdc stop 150
Q-TOF
(a)
g00219bio 1994 3 (0.086) Cu (0.50); Is (1.00,1.00) X1488H4
TOF MS ES+
9.99e12
100
%
0
1075.59
1194.98
(b)
g00219bio 1994 3 8 (0.704) Cm (1:8)
TOF MS ES+
3.47e3
100
%
0
1061.98
1063.78
1095.13
1097.04
1130.41
1132.32
1168.03
1168.74
1173.48
1208.27
1213.89
1060
1070
1080
1090
1100
1110
1120
1130
1140
1150
1160
1170
1180
1190
1200
1210
1220
m/z

Figure 5

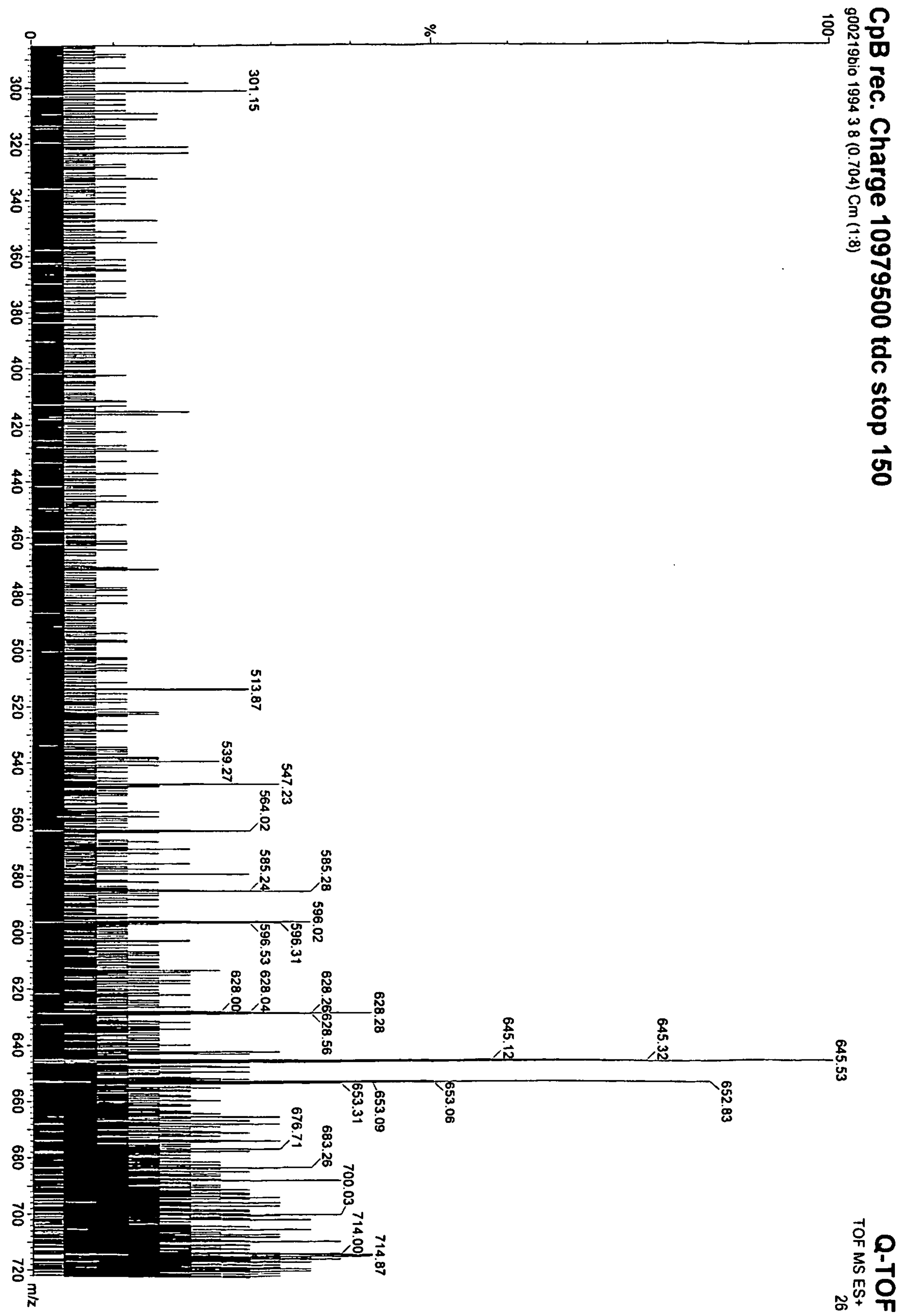
CpB rec. Charge 10979500 tdc stop 150
g0219bio 1994 3 8 (0.704) Cm (1:8)
Q-TOF
TOF MS ES+
26
100
%
0
301.15
513.87
539.27
547.23
564.02
585.24
585.28
596.02
596.31
596.53
628.00
628.04
628.26
628.28
628.56
645.12
645.32
645.53
652.83
653.06
653.09
653.31
676.71
683.26
700.03
714.00
714.87
300
320
340
360
380
400
420
440
460
480
500
520
540
560
580
600
620
640
660
680
700
720
m/z

Figure 6

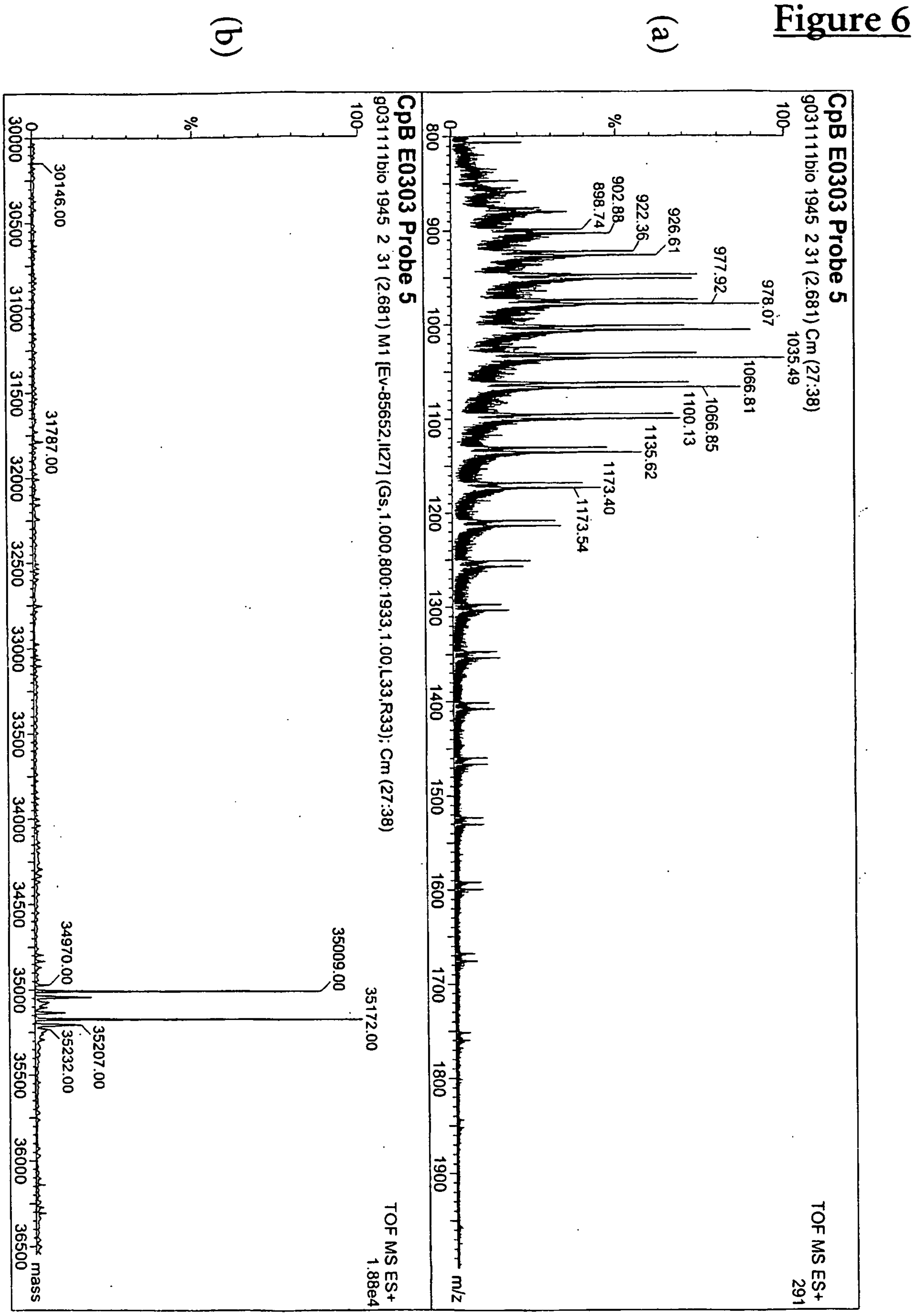
(a)
CpB E0303 Probe 5
g031111bio 1945 2 31 (2.681) Cm (27:38)
TOF MS ES+
291
100
%
0
898.74
902.88
922.36
926.61
977.92
978.07
1035.49
1066.81
1066.85
1100.13
1135.62
1173.40
1173.54
800
900
1000
1100
1200
1300
1400
1500
1600
1700
1800
1900
m/z
(b)
CpB E0303 Probe 5
g031111bio 1945 2 31 (2.681) M1 [Ev-85652,It27] (Gs,1.000,800:1933,1.00,L33,R33); Cm (27:38)
TOF MS ES+
1.88e4
100
%
0
30146.00
31787.00
34970.00
35009.00
35172.00
35207.00
35232.00
30000
30500
31000
31500
32000
32500
33000
33500
34000
34500
35000
35500
36000
36500
mass

Figure 7

(a)

(b)

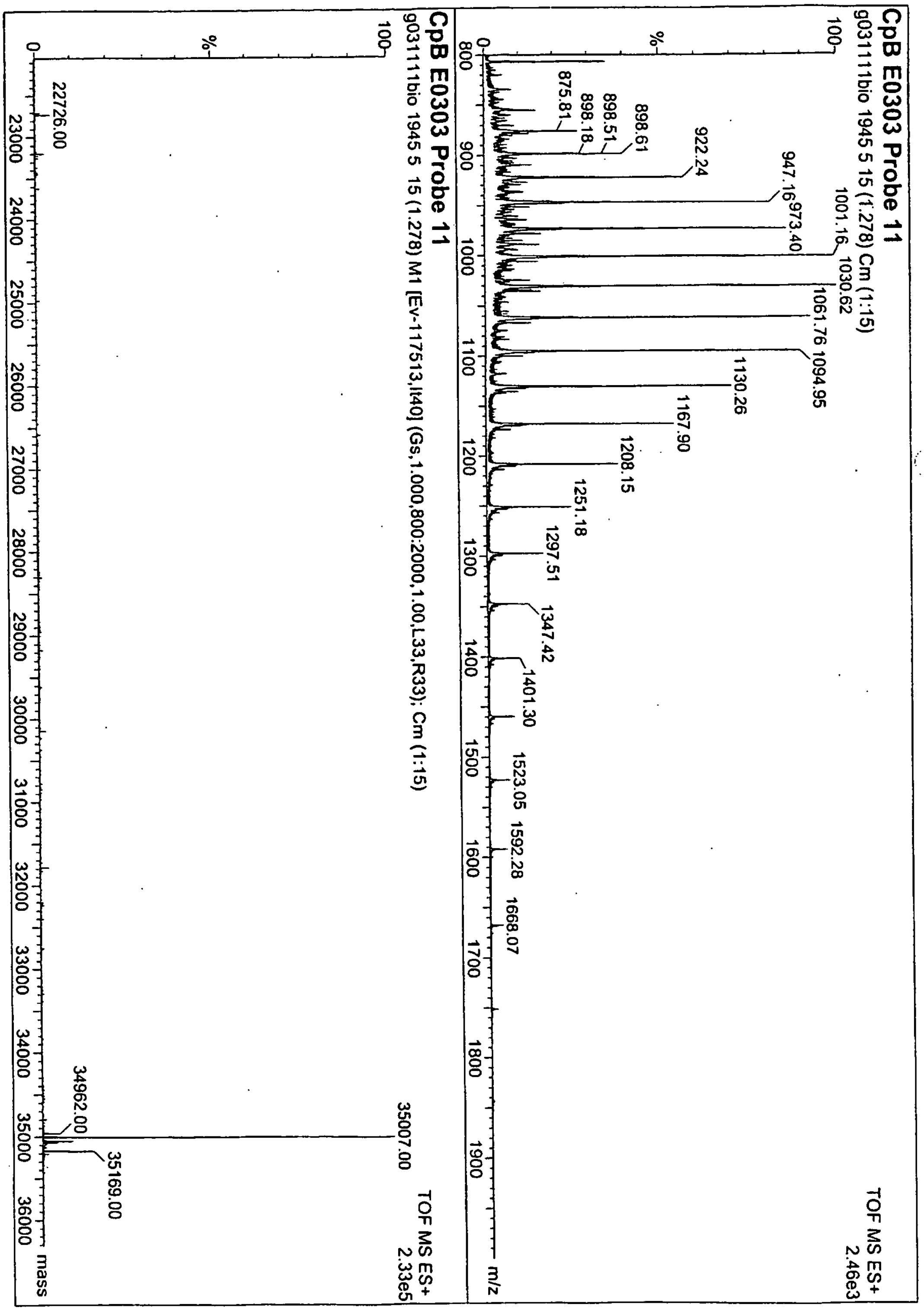
CpB E0303 Probe 11
g031111bio 1945 5 15 (1.278) Cm (1:15)
TOF MS ES+
2.46e3
100
%
0
m/z
875.81
898.18
898.51
898.61
922.24
947.16
973.40
1001.16
1030.62
1061.76
1094.95
1130.26
1167.90
1208.15
1251.18
1297.51
1347.42
1401.30
1523.05
1592.28
1668.07
800
900
1000
1100
1200
1300
1400
1500
1600
1700
1800
1900
CpB E0303 Probe 11
g031111bio 1945 5 15 (1.278) M1 [Ev-117513,It40] (Gs,1.000,800:2000,1.00,L33,R33); Cm (1:15)
TOF MS ES+
2.33e5
100
%
0
mass
22726.00
34962.00
35007.00
35169.00
23000
24000
25000
26000
27000
28000
29000
30000
31000
32000
33000
34000
35000
36000

# Figure 8

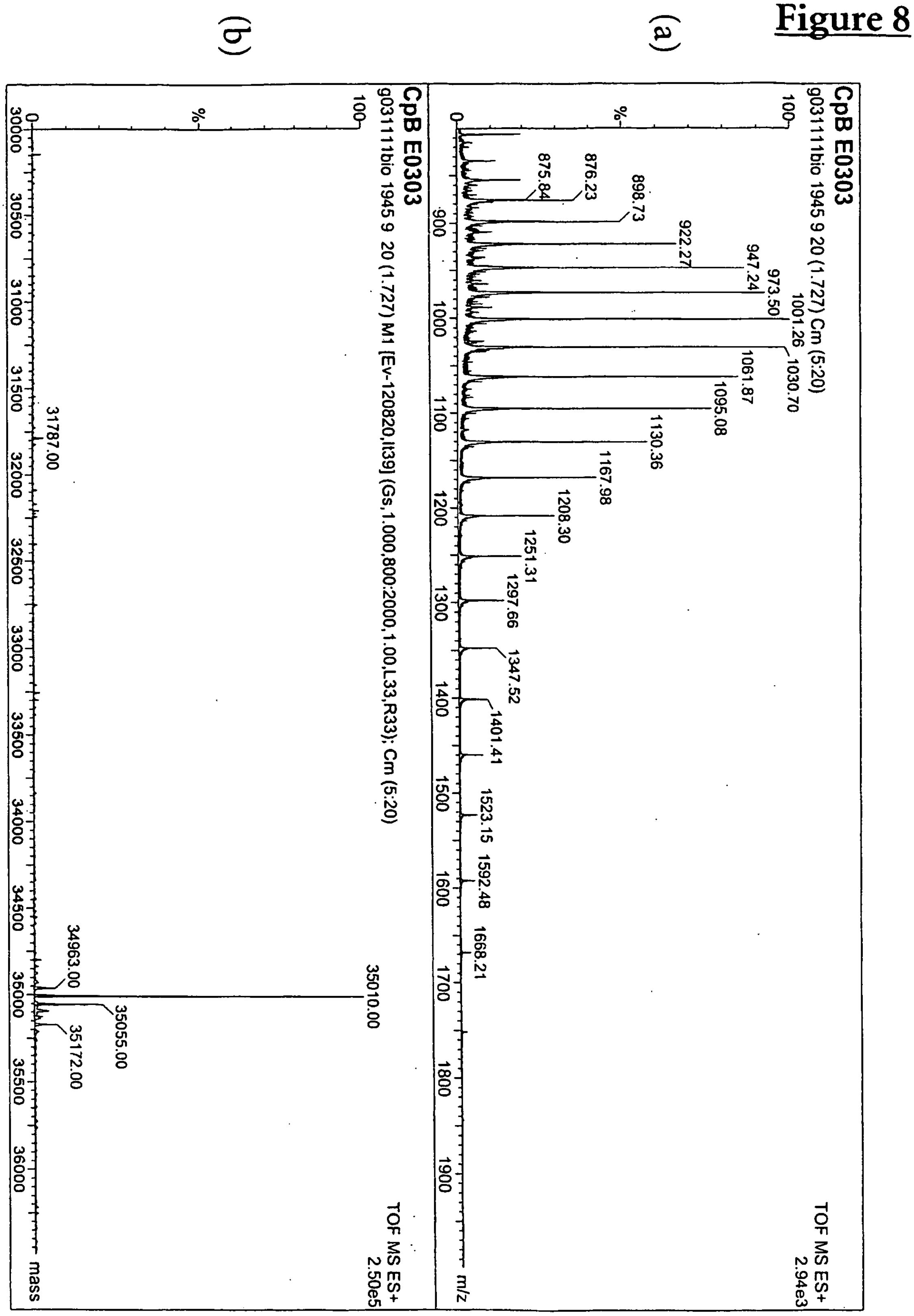
(a)
CpB E0303
g031111bio 1945 9 20 (1.727) Cm (5:20)
TOF MS ES+
2.94e3
100
%
0
875.84
876.23
898.73
922.27
947.24
973.50
1001.26
1030.70
1061.87
1095.08
1130.36
1167.98
1208.30
1251.31
1297.66
1347.52
1401.41
1523.15
1592.48
1668.21
900
1000
1100
1200
1300
1400
1500
1600
1700
1800
1900
m/z
(b)
CpB E0303
g031111bio 1945 9 20 (1.727) M1 [Ev-120820,It39] (Gs,1.000,800:2000,1.00,L33,R33); Cm (5:20)
TOF MS ES+
2.50e5
100
%
0
31787.00
34963.00
35010.00
35055.00
35172.00
30000
30500
31000
31500
32000
32500
33000
33500
34000
34500
35000
35500
36000
mass

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0151624 A
- DE 19915938
- WO 9623064 A
- EP 0253303 A
- EP 0282042 A
- EP 1069131 A
- US 5284933 A
- US 5310663 A
- US 5618676 A
- US 5854018 A
- US 5856123 A
- US 5919651 A
- EP 0116201 A
- US 4870008 A
- US 4683293 A
- US 4808537 A
- US 4812405 A
- US 4818700 A
- US 4837148 A
- US 4855231 A
- US 4857467 A
- US 4879231 A
- US 4882279 A
- US 4885242 A
- US 4895800 A
- US 4929555 A
- US 5002876 A
- US 5004688 A
- US 5032516 A
- US 5122465 A
- US 5135868 A
- US 5166329 A
- WO 0056903 A
- US 5324639 A
- EP 0264250 A
- EP 0195691 A
- EP 03028101 A


### Non-patent literature cited in the description


- Carboxypeptidase B. **Folk J. ; P. Boyer.** The Enzymes 3. Academic Press, 1971, vol. 57
- **Folk, J. ; Gladner, J.** *Biochim Biophys Acta,* 1961, vol. 48, 139-47
- **Zisapel, N. ; Sokolovsky, M.** *Eur J Biochem,* 1975, vol. 54, 541-7
- **Clauser E. et al.** *J. Biol. Chem.,* 1988, vol. 263, 17837-45
- **Ventura et al.** *J. Biol. Chem.,* 1999, vol. 274 (28), 19925-33
- Molecular Biology of the Cell. Garland Science Publishing, 2002
- **Waters et al.** *J. Biol. Chem.,* 1988, vol. 263, 6209-14
- **Porath, J. et al.** *Nature,* 1975, vol. 258, 598-599
- **Hochuli, E. et al.** *J. Chromatogr.,* 1987, vol. 411, 177-184
- **Hochuli, E. et al.** *Bio/Technology,* 1988, vol. 6, 1321-1325
- **Chaga, G. et al.** *Biotechnol. Appl. Biochem.,* 1999, vol. 29, 19-24
- **Chaga, G. et al.** *J. Chromatogr. A,* 1999, vol. 864, 257-256
- **Chen, B.P. ; Hai, T.** *Gene,* 1994, vol. 139, 73-75
- **Rank, K.B. et al.** *Protein Expr. Purif.,* 2001, vol. 22, 258-266
- **Borsing, L. et al.** *Biochem. Biophys. Res. Commun.,* 1997, vol. 240, 586-590
- **Kaslow, D.C. ; Shiloach, J.** *Bio/Technology,* 1994, vol. 12, 494-499
- **Janknecht, R. et al.** *Proc. Natl. Acad. Sci. USA,* 1991, vol. 88, 8972-8976
- **Janknecht, R. ; Nordheim, A.** *Gene,* 1992, vol. 121, 321-324
- **Kuusinen, A. et al.** *Eur. J. Biochem.,* 1995, vol. 233, 720-726
- **Schmidt, M. et al.** *Protein Expr. Purif.,* 1998, vol. 12, 323-330
- **Hakansson, K. et al.** *Acta Crystallogr. D Biol. Crystallogr.,* 2000, vol. 56, 924-926
- **Wu, J. ; Filutowicz, M.** *Acta Biochim. Pol.,* 1999, vol. 46, 591-599
- **Halliwell, C.M. et al.** *Anal Biochem.,* 2001, vol. 295, 257-261
- **Julius, D. et al.** *Cell,* 1984, vol. 37, 1075-1089
- **Werten, M.W. et al.** *Yeast,* 1999, vol. 15, 1087-1096

- **Thill, G.P. et al.** Positive and Negative Effects of Multi-Copy Integrated Expression in Pichia pastoris. *International Symposium on the Genentics of Microorganisms,* 1990, vol. 2, 477-490
- **Vedvick, T. et al.** *J. Ind. Microbiol.,* 1991, vol. 7, 197-201
- **Drocourt, D. et al.** *Nucleic Acids Res.,* 1990, vol. 18, 4009
- **Carmels, T. et al.** *Curr. Genet.,* 1991, vol. 20, 309-314
- **Southern, P.J. ; Berg, P.** *J. Mol. Appl. Genet.,* 1982, vol. 1, 327-341
- **van Treeck, U. et al.** *Antimicrob Agents Chemother.,* 1981, vol. 19, 371-380
- **Beck, E. et al.** *Gene,* 1982, vol. 19, 327-336
- **Chen, X.J. ; Fukuhara, H.** *Gene,* 1988, vol. 69, 181-192
- **Kato, M. et al.** *Eur. J. Biochem,* 2003, vol. 270, 4587-4593
- **Sambrook ; Fritsch ; Maniatis.** Molecular Cloning, A Laboratory Manual. CSHL Press, 2001
- **Higgins, D.R. ; Cregg, J.M.** Pichia Protocols. *Methods in Molecular Biology,* 1998, vol. 103, 107-120
- **Stratton, J. ; Chiruvolu, V. ; Meagher, M.** High cell-density fermentation. *Pichia Fermentation Guidelines, Invitrogen,* 1998
- **Clauser E. et al.** *J. Biol. Chem.,* 1988, vol. 263, 17837-45
- Carboxypeptidase B. **Folk J.** The Enzymes 3. Academic Press, 1971, vol. 57
- **Clauser,E. et al.** *J. Biol. Chem.,* 1988, vol. 263 (33), 17837-17845

SEQUENCE LISTING

<110> Roche Diagnostics GmbH F. Hoffmann-La Roche AG

<120> Recombinantly expressed carboxypeptidase B and purification thereof

<130> 22225

<150> EP 03028101.8

<151> 2003-12-05

<160> 5

<170> PatentIn version 3.2

<210> 1
<211> 1248
<212> DNA
<213> Rattus norvegicus

<220>
<221> misc feature
<223> Rattus norvegicus carboxypeptidase B1 (Cpbl), sequence encoding the pre-pro-enzyme according to Clauser, E. et al (1988) J. Biol. Chem. 263 (33), 17837-17845

<220>
<221> CDS
<222> (1)..(1248)
<223> coding sequence including stop codon

<220>
<221> sig_peptide
<222> (1)..(39)
<223> coding for signal peptide moiety

<220>
<221> misc_feature
<222> (40)..(1248)
<223> coding for pro-carboxypeptidase B (zymogen) moiety

<220>
<221> misc_feature
<222> (325)..(1248)
<223> coding for active carboxypeptidase B fragment

<400> 1

```
atg ttg ctg cta ctg gcc ctg gtg agt gtg gcc ttg gct cat gct tcc       48
Met Leu Leu Leu Leu Ala Leu Val Ser Val Ala Leu Ala His Ala Ser
1               5                   10                  15
```

```
gag gag cac ttt gat ggc aac cgg gtg tac cgt gtc agt gta cat ggt      96
Glu Glu His Phe Asp Gly Asn Arg Val Tyr Arg Val Ser Val His Gly
            20                  25                  30

gaa gat cac gtc aac tta att cag gag cta gcc aac acc aaa gag att     144
Glu Asp His Val Asn Leu Ile Gln Glu Leu Ala Asn Thr Lys Glu Ile
        35                  40                  45

gat ttc tgg aaa cca gat tct gct aca caa gtg aag cct ctc act aca     192
Asp Phe Trp Lys Pro Asp Ser Ala Thr Gln Val Lys Pro Leu Thr Thr
    50                  55                  60

gtt gac ttt cat gtt aaa gca gaa gat gtt gct gat gtg gag aac ttt     240
Val Asp Phe His Val Lys Ala Glu Asp Val Ala Asp Val Glu Asn Phe
65                  70                  75                  80

ctg gag gag aat gaa gtt cac tat gag gta ctg ata agc aac gtg aga     288
Leu Glu Glu Asn Glu Val His Tyr Glu Val Leu Ile Ser Asn Val Arg
                85                  90                  95

aat gct ctg gaa tcc cag ttt gat agc cac acc cgt gca agt gga cac     336
Asn Ala Leu Glu Ser Gln Phe Asp Ser His Thr Arg Ala Ser Gly His
            100                 105                 110

agc tac acc aag tac aac aag tgg gaa acg att gag gcg tgg att caa     384
Ser Tyr Thr Lys Tyr Asn Lys Trp Glu Thr Ile Glu Ala Trp Ile Gln
        115                 120                 125

caa gtt gcc act gat aat cca gac ctt gtc act cag agc gtc att gga     432
Gln Val Ala Thr Asp Asn Pro Asp Leu Val Thr Gln Ser Val Ile Gly
    130                 135                 140

acc aca ttt gaa gga cgt aac atg tat gtc ctc aag att ggc aaa act     480
Thr Thr Phe Glu Gly Arg Asn Met Tyr Val Leu Lys Ile Gly Lys Thr
145                 150                 155                 160

aga ccg aat aag cct gcc atc ttc atc gat tgt ggt ttc cat gca aga     528
Arg Pro Asn Lys Pro Ala Ile Phe Ile Asp Cys Gly Phe His Ala Arg
                165                 170                 175

gag tgg att tct cct gca ttc tgt cag tgg ttt gtg aga gag gct gtc     576
Glu Trp Ile Ser Pro Ala Phe Cys Gln Trp Phe Val Arg Glu Ala Val
            180                 185                 190

cgt acc tat aat caa gag atc cac atg aaa cag ctt cta gat gaa ctg     624
Arg Thr Tyr Asn Gln Glu Ile His Met Lys Gln Leu Leu Asp Glu Leu
        195                 200                 205

gat ttc tat gtt ctg cct gtg gtc aac att gat ggc tat gtc tac acc     672
Asp Phe Tyr Val Leu Pro Val Val Asn Ile Asp Gly Tyr Val Tyr Thr
    210                 215                 220

tgg act aag gac aga atg tgg aga aaa acc cgc tct act atg gct gga     720
Trp Thr Lys Asp Arg Met Trp Arg Lys Thr Arg Ser Thr Met Ala Gly
225                 230                 235                 240
```

```
agt tcc tgc ttg ggt gta aga ccc aac agg aat ttt aat gct ggc tgg      768
Ser Ser Cys Leu Gly Val Arg Pro Asn Arg Asn Phe Asn Ala Gly Trp
                245                 250                 255

tgt gaa gtg gga gct tct cgg agt ccc tgc tct gaa act tac tgt gga      816
Cys Glu Val Gly Ala Ser Arg Ser Pro Cys Ser Glu Thr Tyr Cys Gly
            260                 265                 270

cca gcc cca gag tct gaa aaa gag aca aag gcc ctg gca gat ttc atc      864
Pro Ala Pro Glu Ser Glu Lys Glu Thr Lys Ala Leu Ala Asp Phe Ile
        275                 280                 285

cgc aac aac ctc tcc acc atc aag gcc tac ctg acc atc cac tca tac      912
Arg Asn Asn Leu Ser Thr Ile Lys Ala Tyr Leu Thr Ile His Ser Tyr
    290                 295                 300

tca cag atg atg ctc tac cct tac tcc tat gac tac aaa ctg cct gag      960
Ser Gln Met Met Leu Tyr Pro Tyr Ser Tyr Asp Tyr Lys Leu Pro Glu
305                 310                 315                 320

aac tat gag gaa ttg aat gcc ctg gtg aaa ggt gcg gca aag gag ctt      1008
Asn Tyr Glu Glu Leu Asn Ala Leu Val Lys Gly Ala Ala Lys Glu Leu
                325                 330                 335

gcc act ctg cat ggc acc aag tac aca tat ggc cca gga gct aca aca      1056
Ala Thr Leu His Gly Thr Lys Tyr Thr Tyr Gly Pro Gly Ala Thr Thr
            340                 345                 350

atc tat cct gct gct ggg gga tct gac gac tgg tct tat gat cag gga      1104
Ile Tyr Pro Ala Ala Gly Gly Ser Asp Asp Trp Ser Tyr Asp Gln Gly
        355                 360                 365

atc aaa tat tca ttt acc ttt gaa ctc cgg gat aca ggc ttc ttt ggc      1152
Ile Lys Tyr Ser Phe Thr Phe Glu Leu Arg Asp Thr Gly Phe Phe Gly
    370                 375                 380

ttt ctc ctt cct gag tct cag atc cgc cag acc tgt gag gag aca atg      1200
Phe Leu Leu Pro Glu Ser Gln Ile Arg Gln Thr Cys Glu Glu Thr Met
385                 390                 395                 400

ctt gca gtc aag tac att gcc aat tat gtc cga gaa cat cta tat tag      1248
Leu Ala Val Lys Tyr Ile Ala Asn Tyr Val Arg Glu His Leu Tyr
                405                 410                 415
```

<210> 2
<211> 415
<212> PRT
<213> Rattus norvegicus

<400> 2

Met Leu Leu Leu Leu Ala Leu Val Ser Val Ala Leu Ala His Ala Ser
1               5                   10                  15

Glu Glu His Phe Asp Gly Asn Arg Val Tyr Arg Val Ser Val His Gly
            20                  25                  30

Glu Asp His Val Asn Leu Ile Gln Glu Leu Ala Asn Thr Lys Glu Ile
        35                  40                  45

Asp Phe Trp Lys Pro Asp Ser Ala Thr Gln Val Lys Pro Leu Thr Thr
    50                  55                  60

Val Asp Phe His Val Lys Ala Glu Asp Val Ala Asp Val Glu Asn Phe
65                  70                  75                  80

Leu Glu Glu Asn Glu Val His Tyr Glu Val Leu Ile Ser Asn Val Arg
                85                  90                  95

Asn Ala Leu Glu Ser Gln Phe Asp Ser His Thr Arg Ala Ser Gly His
            100                 105                 110

Ser Tyr Thr Lys Tyr Asn Lys Trp Glu Thr Ile Glu Ala Trp Ile Gln
        115                 120                 125

Gln Val Ala Thr Asp Asn Pro Asp Leu Val Thr Gln Ser Val Ile Gly
    130                 135                 140

Thr Thr Phe Glu Gly Arg Asn Met Tyr Val Leu Lys Ile Gly Lys Thr
145                 150                 155                 160

Arg Pro Asn Lys Pro Ala Ile Phe Ile Asp Cys Gly Phe His Ala Arg
                165                 170                 175

Glu Trp Ile Ser Pro Ala Phe Cys Gln Trp Phe Val Arg Glu Ala Val
            180                 185                 190

Arg Thr Tyr Asn Gln Glu Ile His Met Lys Gln Leu Leu Asp Glu Leu
        195                 200                 205

```
Asp Phe Tyr Val Leu Pro Val Val Asn Ile Asp Gly Tyr Val Tyr Thr
    210                 215                 220

Trp Thr Lys Asp Arg Met Trp Arg Lys Thr Arg Ser Thr Met Ala Gly
225                 230                 235                 240

Ser Ser Cys Leu Gly Val Arg Pro Asn Arg Asn Phe Asn Ala Gly Trp
                245                 250                 255

Cys Glu Val Gly Ala Ser Arg Ser Pro Cys Ser Glu Thr Tyr Cys Gly
            260                 265                 270

Pro Ala Pro Glu Ser Glu Lys Glu Thr Lys Ala Leu Ala Asp Phe Ile
        275                 280                 285

Arg Asn Asn Leu Ser Thr Ile Lys Ala Tyr Leu Thr Ile His Ser Tyr
    290                 295                 300

Ser Gln Met Met Leu Tyr Pro Tyr Ser Tyr Asp Tyr Lys Leu Pro Glu
305                 310                 315                 320

Asn Tyr Glu Glu Leu Asn Ala Leu Val Lys Gly Ala Ala Lys Glu Leu
                325                 330                 335

Ala Thr Leu His Gly Thr Lys Tyr Thr Tyr Gly Pro Gly Ala Thr Thr
            340                 345                 350

Ile Tyr Pro Ala Ala Gly Gly Ser Asp Asp Trp Ser Tyr Asp Gln Gly
        355                 360                 365

Ile Lys Tyr Ser Phe Thr Phe Glu Leu Arg Asp Thr Gly Phe Phe Gly
    370                 375                 380

Phe Leu Leu Pro Glu Ser Gln Ile Arg Gln Thr Cys Glu Glu Thr Met
385                 390                 395                 400

Leu Ala Val Lys Tyr Ile Ala Asn Tyr Val Arg Glu His Leu Tyr
                405                 410                 415
```

<210> 3
<211> 1497
<212> DNA
<213> Artificial

<220>
<223> Artificial sequence encoding pre-pro-carboxypeptidase B polypeptide, optimized for expression in methylotrophic yeast.

<220>
<221> CDS
<222> (1)..(1497)
<223> Coding sequence including stop codons.

<220>
<221> sig_peptide
<222> (1)..(255)
<223> Sequence encoding a Saccharomyces cerevisiae alpha-factor signal peptide sequence which contains a signal peptidase cleavage site.

<220>
<221> misc_feature
<222> (256)..(261)
<223> Sequence encoding a spacer.

<220>
<221> misc_feature
<222> (262)..(288)
<223> Sequence encoding the Histidine-tag, RGSHHHHHH.

<220>
<221> misc_feature
<222> (286)..(570)
<223> Sequence encoding the rat carboxypeptidase B propeptide; the first codon is also part of the Histidine-tag. The sequence incorporates the amino acid exchanges Lys14Asn and Arg142Asp documented in WO 96/23064.

<220>
<221> misc_feature
<222> (571)..(1497)
<223> Sequence encoding the enzyme moiety of rat carboxypeptidase B

<400> 3

```
atg aga ttt cct tca att ttt act gct gtt tta ttc gca gca tcc tcc      48
Met Arg Phe Pro Ser Ile Phe Thr Ala Val Leu Phe Ala Ala Ser Ser
1               5                   10                  15

gca tta gct gct cca gtc aac act aca aca gaa gat gaa acg gca caa      96
Ala Leu Ala Ala Pro Val Asn Thr Thr Thr Glu Asp Glu Thr Ala Gln
            20                  25                  30
```

```
att ccg gct gaa gct gtc atc ggt tac tca gat tta gaa ggg gat ttc      144
Ile Pro Ala Glu Ala Val Ile Gly Tyr Ser Asp Leu Glu Gly Asp Phe
        35                  40                  45

gat gtt gct gtt ttg cca ttt tcc aac agc aca aat aac ggg tta ttg      192
Asp Val Ala Val Leu Pro Phe Ser Asn Ser Thr Asn Asn Gly Leu Leu
    50                  55                  60

ttt ata aat act act att gcc agc att gct gct aaa gaa gaa ggg gta      240
Phe Ile Asn Thr Thr Ile Ala Ser Ile Ala Ala Lys Glu Glu Gly Val
65                  70                  75                  80

tct ctc gag aag aga tcc gct aga ggt tct cac cac cat cac cat cac      288
Ser Leu Glu Lys Arg Ser Ala Arg Gly Ser His His His His His His
                85                  90                  95

gct tct gag gag cac ttc gac ggt aac aga gtt tac aga gtt tct gtt      336
Ala Ser Glu Glu His Phe Asp Gly Asn Arg Val Tyr Arg Val Ser Val
            100                 105                 110

cac ggt gag gac cac gtt aac ttg att caa gag ttg gct aac act aag      384
His Gly Glu Asp His Val Asn Leu Ile Gln Glu Leu Ala Asn Thr Lys
        115                 120                 125

gag att gac ttc tgg aag cca gac tct gct act caa gtt aag cca ttg      432
Glu Ile Asp Phe Trp Lys Pro Asp Ser Ala Thr Gln Val Lys Pro Leu
    130                 135                 140

act act gtt gac ttc cac gtt aag gct gag gac gtt gcc gat gtt gaa      480
Thr Thr Val Asp Phe His Val Lys Ala Glu Asp Val Ala Asp Val Glu
145                 150                 155                 160

aac ttc ttg gag gag aac gag gtt cac tac gaa gtt ttg atc tct aac      528
Asn Phe Leu Glu Glu Asn Glu Val His Tyr Glu Val Leu Ile Ser Asn
                165                 170                 175

gtt cgt aac gct ttg gaa tcc caa ttc gac tct cac act aga gct tct      576
Val Arg Asn Ala Leu Glu Ser Gln Phe Asp Ser His Thr Arg Ala Ser
            180                 185                 190

ggt cac tct tac act aag tac aac aac tgg gag act att gag gct tgg      624
Gly His Ser Tyr Thr Lys Tyr Asn Asn Trp Glu Thr Ile Glu Ala Trp
        195                 200                 205

att caa caa gtt gct act gac aac cca gac ttg gtt act caa tct gtt      672
Ile Gln Gln Val Ala Thr Asp Asn Pro Asp Leu Val Thr Gln Ser Val
    210                 215                 220

att ggt act act ttc gag ggt aga aac atg tac gtt ttg aag att ggt      720
Ile Gly Thr Thr Phe Glu Gly Arg Asn Met Tyr Val Leu Lys Ile Gly
225                 230                 235                 240

aag act aga cca aac aag cca gct att ttc att gac tgt ggt ttc cac      768
Lys Thr Arg Pro Asn Lys Pro Ala Ile Phe Ile Asp Cys Gly Phe His
                245                 250                 255
```

```
gct aga gaa tgg att tcc cca gct ttc tgt caa tgg ttc gtt aga gag        816
Ala Arg Glu Trp Ile Ser Pro Ala Phe Cys Gln Trp Phe Val Arg Glu
            260                 265                 270

gct gtt aga act tac aac caa gag att cac atg aag caa ttg ttg gac        864
Ala Val Arg Thr Tyr Asn Gln Glu Ile His Met Lys Gln Leu Leu Asp
        275                 280                 285

gag ttg gac ttc tac gtt ttg cca gtt gtt aac att gac ggt tac gtt        912
Glu Leu Asp Phe Tyr Val Leu Pro Val Val Asn Ile Asp Gly Tyr Val
    290                 295                 300

tac act tgg act aag gac aga atg tgg aga aag act cgt tcc act atg        960
Tyr Thr Trp Thr Lys Asp Arg Met Trp Arg Lys Thr Arg Ser Thr Met
305                 310                 315                 320

gct ggt tct tct tgc ctt ggt gtc gat cca aat aga aac ttt aac gct       1008
Ala Gly Ser Ser Cys Leu Gly Val Asp Pro Asn Arg Asn Phe Asn Ala
                325                 330                 335

ggt tgg tgt gag gtc ggt gct tct aga tcc cca tgc tct gaa act tac       1056
Gly Trp Cys Glu Val Gly Ala Ser Arg Ser Pro Cys Ser Glu Thr Tyr
            340                 345                 350

tgt ggt cct gct cct gaa tct gaa aag gag act aag gct ttg gct gac       1104
Cys Gly Pro Ala Pro Glu Ser Glu Lys Glu Thr Lys Ala Leu Ala Asp
        355                 360                 365

ttc att aga aac aac ttg tct act att aag gct tac ttg act att cac       1152
Phe Ile Arg Asn Asn Leu Ser Thr Ile Lys Ala Tyr Leu Thr Ile His
    370                 375                 380

tct tac tct caa atg atg ttg tac cca tac tct tac gac tac aag ttg       1200
Ser Tyr Ser Gln Met Met Leu Tyr Pro Tyr Ser Tyr Asp Tyr Lys Leu
385                 390                 395                 400

cca gaa aac tac gag gag ttg aac gct ttg gtt aag ggt gct gct aaa       1248
Pro Glu Asn Tyr Glu Glu Leu Asn Ala Leu Val Lys Gly Ala Ala Lys
                405                 410                 415

gaa ttg gct act ttg cac ggt act aaa tac act tac ggt cca ggt gct       1296
Glu Leu Ala Thr Leu His Gly Thr Lys Tyr Thr Tyr Gly Pro Gly Ala
            420                 425                 430

act act att tac cca gct gct ggt ggt tct gac gac tgg tct tac gac       1344
Thr Thr Ile Tyr Pro Ala Ala Gly Gly Ser Asp Asp Trp Ser Tyr Asp
        435                 440                 445
```

```
caa ggt att aag tac tct ttc act ttc gag ttg aga gat act ggt ttc      1392
Gln Gly Ile Lys Tyr Ser Phe Thr Phe Glu Leu Arg Asp Thr Gly Phe
    450                 455                 460

ttc ggt ttc ttg ttg cct gag tcc caa att aga caa act tgt gag gaa      1440
Phe Gly Phe Leu Leu Pro Glu Ser Gln Ile Arg Gln Thr Cys Glu Glu
465                 470                 475                 480

acc atg ttg gct gtt aag tac att gct aac tac gtt aga gag cac ttg      1488
Thr Met Leu Ala Val Lys Tyr Ile Ala Asn Tyr Val Arg Glu His Leu
                485                 490                 495

tac taa taa                                                          1497
Tyr
```

<210> 4
<211> 497
<212> PRT
<213> Artificial

<220>
<223> Artificial sequence encoding pre-pro-carboxypeptidase B polypeptide, optimized for expression in methylotrophic yeast.

<400> 4

```
Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ser Ala Arg Gly Ser His His His His His His
                85                  90                  95

Ala Ser Glu Glu His Phe Asp Gly Asn Arg Val Tyr Arg Val Ser Val
            100                 105                 110
```

His Gly Glu Asp His Val Asn Leu Ile Gln Glu Leu Ala Asn Thr Lys
115 120 125

Glu Ile Asp Phe Trp Lys Pro Asp Ser Ala Thr Gln Val Lys Pro Leu
130 135 140

Thr Thr Val Asp Phe His Val Lys Ala Glu Asp Val Ala Asp Val Glu
145 150 155 160

Asn Phe Leu Glu Glu Asn Glu Val His Tyr Glu Val Leu Ile Ser Asn
165 170 175

Val Arg Asn Ala Leu Glu Ser Gln Phe Asp Ser His Thr Arg Ala Ser
180 185 190

Gly His Ser Tyr Thr Lys Tyr Asn Asn Trp Glu Thr Ile Glu Ala Trp
195 200 205

Ile Gln Gln Val Ala Thr Asp Asn Pro Asp Leu Val Thr Gln Ser Val
210 215 220

Ile Gly Thr Thr Phe Glu Gly Arg Asn Met Tyr Val Leu Lys Ile Gly
225 230 235 240

Lys Thr Arg Pro Asn Lys Pro Ala Ile Phe Ile Asp Cys Gly Phe His
245 250 255

Ala Arg Glu Trp Ile Ser Pro Ala Phe Cys Gln Trp Phe Val Arg Glu
260 265 270

Ala Val Arg Thr Tyr Asn Gln Glu Ile His Met Lys Gln Leu Leu Asp
275 280 285

Glu Leu Asp Phe Tyr Val Leu Pro Val Val Asn Ile Asp Gly Tyr Val
290 295 300

Tyr Thr Trp Thr Lys Asp Arg Met Trp Arg Lys Thr Arg Ser Thr Met
305 310 315 320

Ala Gly Ser Ser Cys Leu Gly Val Asp Pro Asn Arg Asn Phe Asn Ala
325 330 335

Gly Trp Cys Glu Val Gly Ala Ser Arg Ser Pro Cys Ser Glu Thr Tyr
340 345 350

Cys Gly Pro Ala Pro Glu Ser Glu Lys Glu Thr Lys Ala Leu Ala Asp
355 360 365

Phe Ile Arg Asn Asn Leu Ser Thr Ile Lys Ala Tyr Leu Thr Ile His
370 375 380

Ser Tyr Ser Gln Met Met Leu Tyr Pro Tyr Ser Tyr Asp Tyr Lys Leu
385 390 395 400

Pro Glu Asn Tyr Glu Glu Leu Asn Ala Leu Val Lys Gly Ala Ala Lys
405 410 415

Glu Leu Ala Thr Leu His Gly Thr Lys Tyr Thr Tyr Gly Pro Gly Ala
420 425 430

Thr Thr Ile Tyr Pro Ala Ala Gly Gly Ser Asp Asp Trp Ser Tyr Asp
435 440 445

Gln Gly Ile Lys Tyr Ser Phe Thr Phe Glu Leu Arg Asp Thr Gly Phe
450 455 460

Phe Gly Phe Leu Leu Pro Glu Ser Gln Ile Arg Gln Thr Cys Glu Glu
465 470 475 480

Thr Met Leu Ala Val Lys Tyr Ile Ala Asn Tyr Val Arg Glu His Leu
485 490 495

Tyr

<210> 5
<211> 938
<212> DNA
<213> Pichia pastoris

<220>
<221> promoter
<222> (1)..(938)
<223> Pichia pastoris AOX promoter

<400> 5

```
agatctaaca tccaaagacg aaaggttgaa tgaaaccttt ttgccatccg acatccacag     60
gtccattctc acacataagt gccaaacgca acaggagggg atacactagc agcagaccgt    120
tgcaaacgca ggacctccac tcctcttctc ctcaacaccc acttttgcca tcgaaaaacc    180
agcccagtta ttgggcttga ttggagctcg ctcattccaa ttccttctat taggctacta    240
acaccatgac tttattagcc tgtctatcct ggcccccctg gcgaggttca tgtttgttta    300
tttccgaatg caacaagctc cgcattacac ccgaacatca ctccagatga gggctttctg    360
agtgtggggt caaatagttt catgttcccc aaatggccca aaactgacag tttaaacgct    420
gtcttggaac ctaatatgac aaaagcgtga tctcatccaa gatgaactaa gtttggttcg    480
ttgaaatgct aacggccagt tggtcaaaaa gaaacttcca aaagtcggca taccgtttgt    540
cttgtttggt attgattgac gaatgctcaa aaataatctc attaatgctt agcgcagtct    600
ctctatcgct tctgaacccc ggtgcacctg tgccgaaacg caaatgggga aacacccgct    660
ttttggatga ttatgcattg tctccacatt gtatgcttcc aagattctgg tgggaatact    720
gctgatagcc taacgttcat gatcaaaatt taactgttct aacccctact tgacagcaat    780
atataaacag aaggaagctg ccctgtctta aacctttttt tttatcatca ttattagctt    840
actttcataa ttgcgactgg ttccaattga caagcttttg attttaacga cttttaacga    900
caacttgaga agatcaaaaa acaactaatt attcgaaa                            938
```